# EUROPEAN PATENT APPLICATION

(11) **EP 1 902 732 A1**
(43) Date of publication of application: **26.03.2008**
(21) Application number: 06767652.8
(22) Date of filing: 23.06.2006
(51) Int. Cl.: A61K 45/00, A61K 31/155, A61K 31/352, A61K 31/357, A61K 31/365, A61K 31/7004, A61K 35/12, A61K 35/16, A61K 36/18, A61K 38/00, A61K 39/395, A61P 35/00, A61P 35/02, A61P 35/04, A61P 43/00, C12N 15/09, C12Q 1/02, G01N 33/15, G01N 33/50

(54) **PROPHYLACTIC/THERAPEUTIC AGENT FOR HER2-EXPRESSING CANCER**

(30) Priority: 24.06.2005 JP 2005184483
(71) Applicant: Takeda Pharmaceutical Company Limited, Osaka-shi, Osaka 541-0045 (JP)
(72) Inventor: YOSHIDA, Sei c/o Takeda Pharmaceutical Company Ltd, Tsukuba-shi, Ibaraki 3004293 (JP); HAYASHI, Akira Takeda Pharmaceutical Company Ltd., Tsukuba-shi, Ibaraki 3004293 (JP); NAITO, Kenichiro Takeda Pharmaceutical Company Ltd, Osaka-shi, Osaka; 5328686 (JP); YOSHIMURA, Koji Takeda Pharmaceutical Company Ltd., Tsukuba-shi, Ibaraki; 3004247 (JP); FUJIOKA, Toshiyuki c/o Takeda Pharmaceutical Company Ltd., Osaka-shi, Osaka; 5328686 (JP)
(74) Representative: Kalhammer, Georg
(86) International application number: PCT/JP2006/313035
(87) International publication number: WO 2006/137595

(57) **Abstract**

The present invention provides a prophylactic/therapeutic agent for HER2-expressing cancer, an inhibitor for the proliferation of an HER2-expressing cell, and an energy metabolism inhibitor or an energy metabolism inhibitor mimetic which is useful as an HER2 signal inhibitor.

## Description

### FIELD OF THE INVENTION

The present invention relates to a prophylactic/therapeutic agent for HER2-expressing cancer comprising an energy metabolism inhibitor or an energy metabolism inhibitor mimetic. More particularly, the present invention relates to a an inhibitor for the proliferation of an HER2-expressing cell, an HER2 signal inhibitor or a method for screening a prophylactic/therapeutic agent for HER2-expressing cancer.

### BACKGROUND ART

HER2 (also referred to as erbB2) is a receptor having a tyrosine kinase activity that belongs to the EGF receptor family, and is a factor involved in growth and malignancy of cancer. HER2 is highly expressed in various tumor types including breast cancer, lung cancer, colorectal cancer, pancreatic cancer and ovary cancer, and prognosis of patients expressing HER2 is poor (Seminar in Oncology. Vol. 27, No. 5, 2000, Supplment 9, pp. 13-19). Thus, anti-HER2 therapy against HER2-expressing tumor is expected to suppress tumor growth, and in fact, humanized monoclonal antibodies against HER2 (trastuzumab, herceptin) show clinical effect against chemotherapeutic-agent-resistant metastatic breast cancer highly expressing HER2 (Seminar in Oncology. Vol. 29, No. 3, Supplment 11, 2002, pp. 38-43). Trastuzumab itself has no activity of neutralizing HER2 signal, but it promotes intracellular uptake of HER2 receptor through binding of the tumor cell and the HER2 receptor, by which the number of receptors on the cell surface decreases, resulting in an antitumoral effect (Cancer Immunology Immunotherapy Vol. 37, pp. 255-263, 1993). This effect is limited to tumors highly expressing HER2 (Journal of Clinical Oncology, Vol. 21, No. 15, pp. 2889-2895, 2003). In order to overcome this disadvantage, attempts have been made to neutralize and inhibit HER2 signal, like inhibiting receptor tyrosine kinase for suppressing tumor growth.

On the other hand, cancer cells require a large amount of energy in order to maintain infinite proliferative capacity and metastasizing capacity. ATP is the energy of cells, and in order to supply this energy, cancer cells increase metabolic pathways involved in energy production such as glucose metabolism, lipid metabolism, amino acid metabolism and the like. For example, increase in sugar uptake by a cancer cell is common (Genomics Vol. 84, pp. 1014-1020, 2004, Nature Reviews of Cancer Vol. 4, pp. 891-899, 2004), and this has been the technical foundation for PET diagnosis. In addition to transducing cell proliferation signals, a growth factor receptor directly or indirectly increases a metabolism activity including mitochondria via a signaling molecule such as downstream mTOR, to promote energy metabolism of the receptor-expressing cells (Current Opinion of Clinical Nutrition Metabolism Care, Vol. 8, No. 1, pp. 67-72, 2005).

### DISCLOSURE OF THE INVENTION

There has been a great need for a safe and therapeutically effective prophylactic/therapeutic agent for HER2-expressing cancer.

In order to solve the above problem, the present inventors have gone through keen studies and found that phosphorylation of HER2 is reduced and proliferation of an HER2-expressing cell is inhibited by suppressing energy metabolism or mimicking energy metabolism suppression through glucose metabolism suppression, respiration suppression and mitochondria activity suppression, and with further study accomplished the present invention.

Thus, the present invention provides:
(1) a prophylactic/therapeutic agent for HER2-expressing cancer comprising an energy metabolism inhibitor or an energy metabolism inhibitor mimetic;
(2) the prophylactic/therapeutic agent according to (1) above wherein the energy metabolism inhibitor is an ATP production inhibitor;
(3) the prophylactic/therapeutic agent according to (2) above wherein the ATP production inhibitor is a glucose metabolism inhibitor;
(4) the prophylactic/therapeutic agent according to (3) above wherein the glucose metabolism inhibitor is a mitochondrial activity inhibitor;
(5) the prophylactic/therapeutic agent according to (4) above wherein the mitochondrial activity inhibitor is an electron transport system inhibitor;
(6) the prophylactic/therapeutic agent according to (5) above wherein the electron transport system inhibitor is an agent that inhibits at least one selected from respiratory chain complexes I, II, III, IV and V;
(7) the prophylactic/therapeutic agent according to (4) above wherein the mitochondrial activity inhibitor is a TCA cycle inhibitor;
(8) the prophylactic/therapeutic agent according to (4) above wherein the mitochondrial activity inhibitor is an AMP-activated protein kinase activator;
(9) the prophylactic/therapeutic agent according to (3) above wherein the glucose metabolism inhibitor is a glycolysis inhibitor;
(10) the prophylactic/therapeutic agent according to (1) above wherein the energy metabolism inhibitor mimetic is an AMP-activated protein kinase activator;
(11) a prophylactic/therapeutic agent for HER2-expressing cancer comprising an energy metabolism inhibitor mimetic;
(12) the prophylactic/therapeutic agent according to (11) above wherein the energy metabolism inhibitor mimetic is an AMP-activated protein kinase activator;
(13) the prophylactic/therapeutic agent according to (12) above wherein the AMP-activated protein kinase activator is a mitochondrial activity inhibitor;
(14) the prophylactic/therapeutic agent according to (1) or (11) above wherein HER2 is a protein comprising an amino acid sequence identical to or substantially identical to the amino acid sequence represented by SEQ ID NO: 1, SEQ ID NO: 3 or SEQ ID NO: 5, or a partial peptide or a salt thereof;
(15) the prophylactic/therapeutic agent according to (1) or (11) above which is an inhibitor for proliferation of an HER2-expressing cell and/or an inhibitor for HER2 signal;
(16) the prophylactic/therapeutic agent according to (15) above wherein the signal is at least one selected from proliferation signal, antiapoptotic signal, infiltrative signal and metastasis signal of a cancer cell;
(17) the prophylactic/therapeutic agent according to (1) or (11) above which is a dephosphorylating agent for HER2;
(18) the prophylactic/therapeutic agent according to (1) or (11) above which is a metastasis/recurrence suppressor for cancer or a pro-apoptotic agent for a cancer cell;
(19) a prophylactic/therapeutic agent for HER2-expressing cancer, comprising a substance having a mitochondrial respiratory chain complex inhibiting effect and an AMP-activated protein kinase activating effect;
(20) a method for screening a prophylactic/therapeutic agent for HER2-expressing cancer, comprising bringing a test compound into contact with a cell to determine intracellular energy metabolism;
   (20a) the screening method according to (20) above wherein the indicator of the intracellular energy metabolism is an ATP amount in the cell;
   (20b) the screening method according to (20) above wherein the indicator of the intracellular energy metabolism is a mitochondrial redox activity;
   (20c) the screening method according to (20) above wherein the indicator of the intracellular energy metabolism is phosphorylation of AMP-activated protein kinase (AMPK) protein and/or phosphorylation of AMPK substrate protein,
(21) a method for preventing/treating HER2-expressing cancer, comprising inhibiting energy metabolism or mimicking energy metabolism inhibition;
   (21a) the method according to (21) above wherein the energy metabolism is ATP production;
   (21b) the method according to (21a) above wherein the ATP production is glucose metabolism;
   (21c) the method according to (21b) above wherein the glucose metabolism is a mitochondrial activity;
   (21d) the method according to (21 c) above wherein the mitochondrial activity is an electron transport system;
   (21e) the method according to (21d) above wherein the electron transport system is at least one selected from the respiratory chain complexes I, II, III, IV and V;
   (21f) the method according to (21d) above wherein the mitochondrial activity is TCA cycle;
   (21g) the method according to (21b) above wherein the glucose metabolism is glycolytic;
   (21h) the method according to (21) above wherein the step of mimicking the energy metabolism inhibition is a step of activating AMP-activated protein kinase;
   (21I) a method for preventing/treating HER2-expressing cancer comprising mimicking energy metabolism inhibition;
   (21j) the method according to (21I) above wherein the step of mimicking energy metabolism inhibition is a step of activating AMP-activated protein kinase;
   (21k) the method according to (21j) above wherein the step of activating AMP-activated protein kinase is a step of inhibiting mitochondrial activity;
(22) a method for preventing/treating HER2-expressing cancer, comprising administering an effective amount of an energy metabolism inhibitor or an energy metabolism inhibitor mimetic to a mammal;
(23) use of an energy metabolism inhibitor or an energy metabolism inhibitor mimetic for the manufacture of a prophylactic/therapeutic agent for HER2-expressing cancer; and
(24) a method for preventing/treating HER2-expressing cancer, comprising administering an energy metabolism inhibitor or an energy metabolism inhibitor mimetic to a patient expressing HER2 and having increased glucose metabolism.

The present invention also provides:
(i) a prophylactic/therapeutic agent for a disease associated with HER2, comprising an energy metabolism inhibitor or an energy metabolism inhibitor mimetic; and
(ii) the prophylactic/therapeutic agent described in (i) above wherein the disease associated with HER2 is cancer or rheumatoid arthritis.

Since the energy metabolism inhibitor or the energy metabolism inhibitor mimetic suppresses HER2 phosphorylation, inhibits HER2 signal, and inhibits proliferation of an HER2-expressing cell, it may be used as a safe drug such as a prophylactic/therapeutic agent for HER2-expressing cancer, an inhibitor for the proliferation of an HER2-expressing cell, an HER2 signal inhibitor, a dephosphorylating agent for HER2, a metastasis/recurrence suppressor for cancer, a pro-apoptotic agent for a cancer cell or a prophylactic/therapeutic agent for rheumatoid arthritis. Furthermore, HER2-expressing cancer can be prevented/treated by administering the energy metabolism inhibitor or the energy metabolism inhibitor mimetic to a patient suffering from HER2-expressing cancer. A prophylactic/therapeutic agent for HER2-expressing cancer can be screened efficiently by bringing a test compound into contact with a cell to determine intracellular energy metabolism.

### BRIEF DESCRIPTION OF THE DRAWINGS

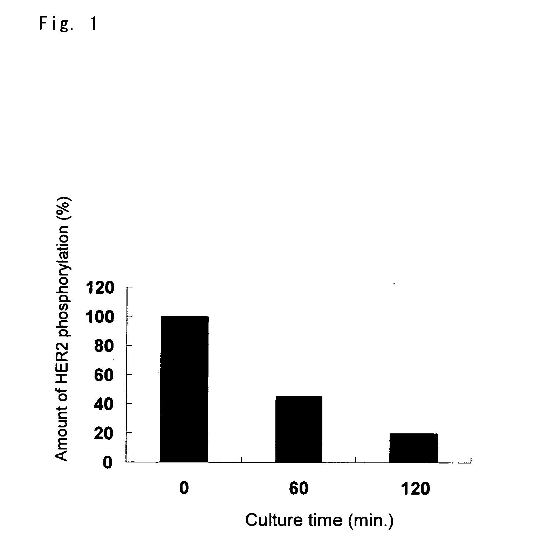

Figure 1 shows the result of determining the change in the amount of HER2 phosphorylation over time while decreasing the oxygen concentration from 20% to 7% upon cell culture. In the figure, the vertical axis represents the amount of phosphorylation of HER2 protein at each time point relative to the amount of phosphorylation of HER2 protein cultured with an oxygen concentration of 20% (100%).

### BEST MODES FOR CARRYING OUT THE INVENTION

### (1) Prophylactic/therapeutic agent for HER2-expressing cancer comprising energy metabolism inhibitor or energy metabolism inhibitor mimetic

The present invention is based on the finding that proliferation of an HER2-expressing cell is significantly inhibited by suppressing energy metabolism or by mimicking energy metabolism suppression of the cell. Thus, in one aspect, the present invention provides a prophylactic/therapeutic agent for HER2-expressing cancer comprising an energy metabolism inhibitor or an energy metabolism inhibitor mimetic.

The "energy metabolism inhibitor" comprises a substance having an effect of inhibiting energy metabolism of a cell (e.g., a synthetic compound, a peptide, a protein, an antibody, a non-peptide compound, a fermented product, a cell extract, a plant extract, an animal tissue extract, plasma, etc.), which may be by itself or contained in an agent. The energy metabolism of a cell is directly or indirectly associated with ATP production, a transporter, AMP-activated protein kinase or the like, and thus an "energy metabolism inhibitor" typically comprises, for example, an ATP production inhibitor, an inhibitor of transporter activity or the like.

The "cell" includes, for example, animal cells (e.g., mammal cells), insect cells and the like. Preferably, the cell is an animal cell, more preferably a human cell, and most preferably a human cancer cell, particularly an HER2-expressing cell.

The "ATP production inhibitor" may be a substance having an effect of inhibiting ATP production (e.g., a synthetic compound, a peptide, a protein, an antibody, a non-peptide compound, a fermented product, a cell extract, a plant extract, an animal tissue extract, plasma, etc.), which may be by itself or contained in an agent, for example, a glucose metabolism inhibitor, an amino acid metabolism inhibitor, a lipid metabolism inhibitor or the like.

The "glucose metabolism inhibitor" may be a substance having an effect of inhibiting glucose metabolism (e.g., a synthetic compound, a peptide, a protein, an antibody, a non-peptide compound, a fermented product, a cell extract, a plant extract, an animal tissue extract, plasma, etc.), which may be by itself or contained in an agent, for example, a mitochondrial activity inhibitor, a glycolysis inhibitor or the like.

The "mitochondrial activity inhibitor" may be a substance having an effect of inhibiting mitochondrial activity and function (e.g., a synthetic compound, a peptide, a protein, an antibody, a non-peptide compound, a fermented product, a cell extract, a plant extract, an animal tissue extract, plasma, etc.), which may be by itself or contained in an agent, for example, an electron transport system inhibitor, a TCA cycle inhibitor or the like. In addition, suppression of aerobic metabolism by, for example, induction of low oxygen state or restriction of a nutrient supply such as sugar, lipid, amino acid or the like can be considered equivalent to the use of a mitochondrial activity inhibitor.

The "electron transport system inhibitor" may be a substance having an effect of inhibiting an electron transport system (e.g., a synthetic compound, a peptide, a protein, an antibody, a non-peptide compound, a fermented product, a cell extract, a plant extract, an animal tissue extract, plasma, etc.), which may be by itself or contained in an agent, for example, an agent that inhibits at least one selected from respiratory chain complexes I, II, III, IV and V.

The "agent that inhibits at least one selected from respiratory chain complexes I, II, III, IV and V" may be a substance having an effect of inhibiting at least one selected from respiratory chain complexes I, II, III, IV and V (e.g., a synthetic compound, a peptide, a protein, an antibody, a non-peptide compound, a fermented product, a cell extract, a plant extract, an animal tissue extract, plasma, etc.), which may be by itself or contained in an agent, for example, rotenone, piericidin, myxothiazol, capsaicin, acetogenin, arachidonic acid or the like for complex I; malonic acid, dimethylsuccinate, TTFA (2-Thenoyltrifluoroacetone) or the like for complex II; antimycin, stigmatellin, arachidonic acid or the like for complex III; sodium azide, cyanide, hydrogen sulfide, hydrocortisone, dexamethasone or the like for complex IV; 2,4-dinitrophenol, valinomycin or oligomycin for complex V; FCCP (carbonyl cyanide-4-(trifluoromethoxy) phenylhydrazone) or the like.

The "TCA cycle inhibitor" may be a substance having an effect of inhibiting TCA cycle (e.g., a synthetic compound, a peptide, a protein, an antibody, a non-peptide compound, a fermented product, a cell extract, a plant extract, an animal tissue extract, plasma, etc.), which may be by itself or contained in an agent, for example, trifluoroacetic acid, salicylic acid or the like.

The "glycolysis inhibitor" may be a substance having an effect of inhibiting glycolysis (e.g., a synthetic compound, a peptide, a protein, an antibody, a non-peptide compound, a fermented product, a cell extract, a plant extract, an animal tissue extract, plasma, etc.), which may be by itself or contained in an agent, for example, 2-deoxyglucose, sodium fluoride, lithium iodoacetate or the like.

The "amino acid metabolism inhibitor" may be a substance having an effect of inhibiting amino acid metabolism (e.g., a synthetic compound, a peptide, a protein, an antibody, a non-peptide compound, a fermented product, a cell extract, a plant extract, an animal tissue extract, plasma, etc.), which may be by itself or contained in an agent, for example, a transamination inhibitor (e.g., aminooxyacetyl acid, etc.), a deamination inhibitor, a decarboxylation inhibitor or the like.

The "lipid metabolism inhibitor" may be a substance having an effect of inhibiting lipid metabolism (e.g., a synthetic compound, a peptide, a protein, an antibody, a non-peptide compound, a fermented product, a cell extract, a plant extract, an animal tissue extract, plasma, etc.), which may be by itself or contained in an agent, for example, a beta-oxidation inhibitor (e.g., tetradecyglycidic acid, etc.).

The "inhibitor of transporter activity" may be a substance having an effect of inhibiting a transporter activity of a cell (e.g., a synthetic compound, a peptide, a protein, an antibody, a non-peptide compound, a fermented product, a cell extract, a plant extract, an animal tissue extract, plasma, etc.), which may be by itself or contained in an agent. Examples of the "inhibitor of transporter activity" include but not limited to a glucose transporter inhibitor (e.g., phlorizin, etc.), a pyruvic acid-lactic acid transporter inhibitor (e.g., alpha-cyano-4-hydroxycinnamate, p-chloromercuribenzenesulfonic acid, etc.) and an amino acid transporter inhibitor (e.g., 2-aminobicyclo heptane carboxylic acid (2-aminobicyclo-(2,2,1)-heptane-2-carboxylic acid (BCH)), etc.), and include any substance that is capable of inhibiting energy metabolism of a cell by inhibiting a transporter activity to suppress a supply of a substrate necessary for energy metabolism of the cell.

The "energy metabolism inhibitor mimetic" may be a substance that induces a physiologic response of a cell resulting from an inhibited or starved state of energy metabolism (e.g., phosphorylation and dephosphorylation of a protein, change in an amount of active oxygen production, change in an enzymatic activity, change in mRNA transcription-translation, change in protein degradation and synthesis, etc.) without directly inhibiting energy metabolism of the cell (e.g., a synthetic compound, a peptide, a protein, an antibody, a non-peptide compound, a fermented product, a cell extract, a plant extract, an animal tissue extract, plasma, etc.), which may be by itself or contained an agent, for example, an AMP-activated protein kinase activator.

The "AMP-activated protein kinase activator" may be a substance having an effect of activating AMP-activated protein kinase (e.g., a synthetic compound, a peptide, a protein, an antibody, a non-peptide compound, a fermented product, a cell extract, a plant extract, an animal tissue extract, plasma, etc.), which may be by itself or contained in an agent. Examples include but not limited to metformin, phenformin, buformin, AICAR (5-aminoimidazole-4-carboxamide ribonucleoside), an insulin sensitizer (e.g., troglitazone, pioglitazone or a salt thereof (preferably, hydrochloride), rosiglitazone or a salt thereof (preferably, maleate), reglixane, netoglitazone, rivoglitazone, edaglitazone, tesaglitazar, ragaglitazar, muraglitazar, metaglidasen, naveglitazar, balaglitazone, etc.), and include any substance that activates AMP-activated protein kinase to suppress HER2 phosphorylation. The "AMP-activated protein kinase activator" may have the effect as the "mitochondrial activity inhibitor" mentioned above.

HER2 (hereinafter, also referred to as a protein of the present invention or a protein used with the present invention) may be a cell (e.g., a retinal cell, a hepatic cell, a spleen cell, a nerve cell, a glial cell, a pancreatic β cell, a myeloid cell, a mesangial cell, a Langerhans cell, an epidermal cell, an epithelial cell, an endothelial cell, a fibroblast cell, a fiber cell, a muscle cell, an adipocyte, an immunocyte (e.g., macrophage, a T cell, a B cell, a natural killer cell, a mast cell, a neutrophil cell, a basophil cell, an eosinophilic cell, a monocyte, a platelet, etc.), a megakaryocyte, a synovial cell, a chondrocyte, a bone cell, an osteoblast, an osteoclast, a mammary cell, a hepatic cell or a stroma cell, or a precursor cell, a stem cell or a cancer cell of these cells, etc.) from a human or warm-blooded animal (e.g., guinea pig, rat, mouse, chicken, rabbit, pig, sheep, cow, monkey or the like), any tissue where these cells are present, for example, brain, a site of brain (e.g., retina, olfactory bulb, amygdaloid nuclear, basal ganglia, hippocampus, thalamus, hypothalamus, cerebral cortex, medulla or cerebellum), spinal marrow, pituitary gland, stomach, pancreas, kidney, liver, genital gland, thyroid gland, gallbladder, bone marrow, adrenal gland, skin, muscle, lung, digestive tract (e.g., large intestine or small intestine), blood vessel, heart, thymus gland, spleen, submaxillary gland, peripheral blood, prostate gland, testicle, ovary, placenta, uterus, bone, joint, skeletal muscle or the like, a protein derived from a blood cell or a cultured cell thereof (e.g., MEL, M1, CTLL-2, HT-2, WEHI-3, HL-60, JOSK-1, K562, ML-1, MOLT-3, MOLT-4, MOLT-10, CCRF-CEM, TALL-1, Jurkat, CCRT-HSB-2, KE-37, SKW-3, HUT-78, HUT-102, H9, U937, THP-1, HEL, JK-1, CMK, KO-812, MEG-01, etc.), or a synthetic protein.

Examples of HER2 include a protein containing an amino acid sequence identical or substantially identical to the amino acid sequence represented by SEQ ID NO: 1, SEQ ID NO: 3 or SEQ ID NO: 5, and a partial peptide and a salt thereof.

Examples of amino acid sequences substantially identical to the amino acid sequence represented by SEQ ID NO: 1, SEQ ID NO: 3 or SEQ ID NO: 5 include amino acid sequences having about 50% or higher, preferably about 60% or higher, more preferably about 70% or higher, more preferably about 80% or higher, particularly preferably about 90% or higher, most preferably about 95% or higher homology with the amino acid sequence represented by SEQ ID NO: 1, SEQ ID NO: 3 or SEQ ID NO: 5.

Homology of an amino acid sequence can be calculated using homology calculation algorithm NCBI BLAST (National Center for Biotechnology Information Basic Local Alignment Search Tool) under the following conditions (expect value = 10; gap penalty; matrix = BLOSUM62; filtering = OFF).

A protein containing an amino acid sequence substantially identical to the amino acid sequence represented by SEQ ID NO: 1, SEQ ID NO: 3 or SEQ ID NO: 5 may include, for example, a protein containing an amino acid sequence substantially identical to the amino acid sequence represented by SEQ ID NO: 1, SEQ ID NO: 3 or SEQ ID NO: 5, and having a substantially identical activity to the protein containing the amino acid sequence represented by SEQ ID NO: 1, SEQ ID NO: 3 or SEQ ID NO: 5.

An example of a substantially identical activity includes HER2 action and the like. The phrase "substantially identical" means that their properties are identical in nature (e.g., in physiological or pharmacological way). Thus, HER2 action or the like are preferably equivalent (e.g., about 0.01-100 times, preferably about 0.1-10 times, more preferably 0.5-2 times), while the quantitative factors such as the level of these activities, the molecular weight of the protein or the like may be different.

Furthermore, examples of the proteins of the present invention also include so-called muteins such as proteins containing (i) an amino acid sequence having a single or two or more (e.g., approximately 1-100, preferably approximately 1-30, preferably approximately 1-10, more preferably several (1-5)) amino acids deleted from the amino acid sequence represented by SEQ ID NO: 1, SEQ ID NO: 3 or SEQ ID NO: 5, (ii) an amino acid sequence having a single or two or more (e.g., approximately 1-100, preferably approximately 1-30, preferably approximately 1-10, more preferably several (1-5)) amino acids added to the amino acid sequence represented by SEQ ID NO: 1, SEQ ID NO: 3 or SEQ ID NO: 5, (iii) an amino acid sequence having a single or two or more (e.g., approximately 1-100, preferably approximately 1-30, preferably approximately 1-10, more preferably several (1-5)) amino acids inserted into the amino acid sequence represented by SEQ ID NO: 1, SEQ ID NO: 3 or SEQ ID NO: 5, (iv) an amino acid sequence having a single or two or more (e.g., approximately 1-100, preferably approximately 1-30, preferably approximately 1-10, more preferably several (1-5)) amino acids in the amino acid sequence represented by SEQ ID NO: 1, SEQ ID NO: 3 or SEQ ID NO: 5 substituted for other amino acids, or (v) an amino acid sequence obtained by a combination of above.

The positions of the insertion, deletion or substitution in the amino acid sequence mentioned above are not particularly limited.

In accordance with customary representation, proteins in the present specification have its N-terminal (amino terminal) at the left end and C-terminal (carboxyl terminal) at the right end. Proteins used with the present invention including proteins containing the amino acid sequence represented by SEQ ID NO: 1 may have any of carboxyl group (-COOH), carboxylate (-COO⁻), amide (-CONH₂) or ester (-COOR) as C-terminal.

Herein, R in ester may be, for example, C₁₋₆ alkyl group such as methyl, ethyl, n-propyl, isopropyl or n-butyl, for example, C₃₋₈ cycloalkyl group such as cyclopentyl or cyclohexyl, for example, C₆₋₁₂ aryl group such as phenyl or α-naphthyl, for example, C₇₋₁₄ aralkyl group such as phenyl-C₁₋₂ alkyl group, e.g., benzyl or phenethyl or α-naphthyl-C₁₋₂ alkyl group, e.g., α-naphthylmethyl, or pivaloyloxy methyl group.

When the protein used with the present invention has carboxyl group (or carboxylate) at a position other than the C-terminal, amidated or esterified carboxyl group may be contained in the protein used with the present invention. Ester in this case may be, for example, ester at the C-terminal described above.

Proteins used with the present invention also include those in which an amino group of an N-terminal amino acid residue (e.g., methionine residue) is protected by a protecting group (e.g., C₁₋₆ acyl group such as C₁₋₆ alkanoyl, for example, formyl group or acetyl group), those in which a glutamine residue produced by cleavage of N-terminal *in vivo* is pyroglutamine-oxidized, those in which a substituent (e.g., -OH, -SH, amino group, imidazole group, indole group, guanidino group, etc.) on a side chain of an intramolecular amino acid is protected by an appropriate protecting group (e.g., C₁₋₆ acetyl group such as C₁₋₆ alkanoyl group, for example, formyl group or acyl group), or complex proteins such as so-called glycoproteins that have gone through glycosylation.

Specific examples of the proteins used with the present invention include a protein containing the amino acid sequence represented by SEQ ID NO: 1, a protein containing the amino acid sequence represented by SEQ ID NO: 3, and a protein containing the amino acid sequence represented by SEQ ID NO: 5.

A partial peptide of the protein used with the present invention may be a partial peptide of the above-mentioned proteins used with the present invention, and preferably a partial peptide having a similar property to that of the above-mentioned proteins used with the invention.

For example, a peptide having at least 20 or more, preferably 50 or more, more preferably 70 or more, more preferably 100 or more, most preferably 200 or more amino acids of the structural amino acid sequence of the protein used with the present invention can be used.

Furthermore, the partial peptide used with the present invention may have a single or two or more (preferably approximately 1-20, more preferably approximately 1-10, more preferably several (1-5)) amino acids deleted from the amino acid sequence, may have a single or two or more (preferably approximately 1-20, more preferably approximately 1-10, more preferably several (1-5)) amino acids added to the amino acid sequence, may have a single or two or more (preferably approximately 1-20, more preferably approximately 1-10, more preferably several (1-5)) amino acids inserted into the amino acid sequence, or may have a single or two or more (preferably approximately 1-20, more preferably approximately 1-10, more preferably several (1-5)) amino acids in the amino acid sequence substituted for other amino acids.

In addition, the C-terminal of the partial peptide used with the present invention may be any of carboxyl group (-COOH), carboxylate (-COO⁻), amide (-CONH₂) or ester (-COOR).

Similar to the above-mentioned protein used with the present invention, the partial peptides used with the present invention also include those that include carboxyl group (or carboxylate) at a position other than the C-terminal, those in which an amino group of the N-terminal amino acid residue (e.g., methionine residue) is protected by a protecting group, those in which a glutamine residue produced by cleavage of N-terminal *in vivo* is pyroglutamine-oxidized, those in which a substituent on a side chain of an intramolecular amino acid is protected by an appropriate protecting group, or complex proteins such as so-called glycoproteins that have gone through glycosylation.

The partial peptides used with the present invention may also be used as antigens for producing antibodies.

Salts of the proteins or the partial peptides used with the present invention include salts with physiologically acceptable acids (e.g., inorganic acid, organic acid) or bases (e.g., alkali metal salt), particularly preferably physiologically acceptable acid addition salts. Examples of such salts include salts with inorganic acids (e.g., hydrochloric acid, phosphoric acid, hydrobromic acid, sulfuric acid), or salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid).

The proteins, the partial peptides thereof or the salts thereof used with the present invention may be produced from the above-described human or warm-blooded animal cells or tissues by a protein purification method known per se, or may be produced by culturing a transformant containing DNA coding for the protein. Alternatively, it may be produced according to a known method for synthesizing a peptide.

When the protein, the partial peptide thereof or the salt thereof is produced from a human or mammal tissue or cell, the human or mammal tissue or cell can be homogenized, extracted with an acid or the like, and the resulting extract can be purified and isolated by a combination of chromatographies such as reverse phase chromatography and ion-exchange chromatography.

Polynucleotides coding for the protein used with the present invention may be any polynucleotide as long as it contains a nucleotide sequence coding for the protein used with the present invention. Preferably, it is DNA. DNA may be any of genomic DNA, genomic DNA library, cDNA derived from the cells/tissues mentioned above, cDNA library derived from the cells/tissues mentioned above or synthetic DNA.

The vector used for the library may be any of bacteriophage, plasmid, cosmid, phagemid or else. Also, amplification can be performed directly by Reverse Transcription Polymerase Chain Reaction (hereinafter, simply referred to as RT-PCR method) using total RNA or mRNA fraction prepared from the above-mentioned cells/tissues.

Examples of DNA coding for the protein used with the present invention include DNA containing the nucleotide sequence represented by SEQ ID NO: 2, SEQ ID NO: 4 or SEQ ID NO: 6, or any DNA containing a nucleotide sequence that hybridizes with the nucleotide sequence represented by SEQ ID NO: 2, SEQ ID NO: 4 or SEQ ID NO: 6 under highly stringent conditions and that codes for a protein having a property substantially identical to that of a protein including the amino acid sequence represented by SEQ ID NO: 1, SEQ ID NO: 3 or SEQ ID NO: 5.

Examples of DNA that can hybridize with the nucleotide sequence represented by SEQ ID NO: 2, SEQ ID NO: 4 or SEQ ID NO: 6 under highly stringent conditions include DNA containing a nucleotide sequence having about 50% or higher, preferably about 60% or higher, more preferably about 70% or higher, more preferably about 80% or higher, particularly preferably about 90% or higher, and most preferably about 95% or higher homology with the nucleotide sequence represented by SEQ ID NO: 2, SEQ ID NO: 4 or SEQ ID NO: 6.

Homology of nucleotide sequences can be calculated by employing homology calculation algorithm NCBI BLAST (National Center for Biotechnology Information Basic Local Alignment Search Tool) under the following conditions (expect value =10; gap penalty; filtering = ON; match score = 1; mismatch score = -3).

Hybridization can be performed according to a method known per se or a similar method thereto, for example, a method described in Molecular Cloning 2nd (J. Sambrook et al., Cold Spring Harbor Lab. Press, 1989). Furthermore, when a commercially available library is used, hybridization can be performed following the method described in the attached instruction. More preferably, hybridization can be performed under highly stringent conditions.

Highly stringent conditions mean, for example, conditions where the sodium concentration is about 19-40 mM, preferably about 19-20 mM and the temperature is about 50-70°C, preferably about 60-65°C. In particular, the most preferable conditions are where the sodium concentration is about 19 mM and the temperature is about 65°C.

More specifically, DNA coding for a protein including the amino acid sequence represented by SEQ ID NO: 1 may be DNA including the nucleotide sequence represented by SEQ ID NO: 2, DNA coding for a protein including the amino acid sequence represented by SEQ ID NO: 3 may be DNA including the nucleotide sequence represented by SEQ ID NO: 4, and DNA coding for a protein including the amino acid sequence represented by SEQ ID NO: 5 may be DNA including the nucleotide sequence represented by SEQ ID NO: 6.

DNA coding for the partial peptide used with the present invention may be any DNA that includes a nucleotide sequence coding for the above-mentioned partial peptide used with the invention. In addition, it may be any of genomic DNA, genomic DNA library, cDNA derived from the above-mentioned cell/tissue, cDNA library derived from the above-mentioned cell/tissue, or synthetic DNA.

Examples of DNA coding for the partial peptide used with the present invention include DNA having a part of DNA containing the nucleotide sequence represented by SEQ ID NO: 2, SEQ ID NO: 4 or SEQ ID NO: 6, or DNA including a part of DNA containing a nucleotide sequence that hybridizes with the nucleotide sequence represented by SEQ ID NO: 2, SEQ ID NO: 4 or SEQ ID NO: 6 under highly stringent conditions and that codes for a protein having a substantially identical property to that of the protein of the invention.

DNA that can hybridize with the nucleotide sequence represented by SEQ ID NO: 2, SEQ ID NO: 4 or SEQ ID NO: 6 is equivalent to those described above.

A hybridization method and highly stringent conditions used are the same as those described above.

As a process for cloning DNA that codes completely for the protein or the partial peptide used with the present invention (hereinafter, in some cases, simply referred to as the protein of the invention when describing cloning and expression of DNA coding for these proteins and the partial peptides), a synthetic DNA primer having a part of a nucleotide sequence coding for the protein of the present invention can be used for amplification by PCR method, or DNA incorporated in a suitable vector labeled with a DNA fragment or synthetic DNA coding for a part or whole protein of the invention can be used for hybridization. The hybridization method can be carried out, for example, according to Molecular Cloning 2nd Ed (J. Sambrook et al., Cold Spring Harbor Lab. Press, 1989). Furthermore, when a commercially available library is used, hybridization can be carried out according to the method described in the attached instruction.

A nucleotide sequence of DNA can be converted by PCR, with a known kit such as Mutan^{™}-super Express Km (Takara Shuzo Co., Ltd.), Mutan^{™}-K (Takara Shuzo Co., Ltd.) or the like, or by a method known per se such as ODA-LA PCR method, Gapped duplex method, Kunkel method, or a method similar thereto.

DNA coding for a cloned protein may be used directly or, if desired, after digesting with a restriction enzyme or attaching a linker thereto. This DNA has ATG as a translation initiation codon at the 5'-terminal end and TAA, TGA or TAG as a translation stop codon at the 3'-terminal end. The translation initiation codon and the translation stop codon can be attached using a suitable synthetic DNA adaptor.

An expression vector of the protein of the invention can be produced by a known method, for example, (i) by excising a DNA fragment of interest from DNA coding for the protein of the invention and (ii) ligating the DNA fragment downstream from the promoter in a suitable expression vector.

"HER2-expressing cancer" may be of any cancer cell as long as it is expressing HER2 (preferably, a protein containing an amino acid sequence identical to or substantially identical to the amino acid sequence represented by SEQ ID NO: 1, SEQ ID NO: 3 or SEQ ID NO: 5, or a partial peptide thereof). Examples of such cancer cells include cancer cells from which HER2 gene, HER2 messenger RNA or HER2 protein can be detected by a known method such as a gene detection method (e.g., FISH (fluorescence *in situ* hybridization method), CISH (chromosome *in situ* hybridization method), PCR (polymerase chain reaction method), Southern blotting, etc.), a messenger RNA detection method (e.g., RT-PCR (Reverse Transcriptase PCR), ISH *(in situ* hybridization method), Northern blotting, etc.), a protein detection method (e.g., IHC (immuno-histo-chemistry method), western blotting, ELISA (Enzyme-linked immunesorbent assay), etc.) or the like.

HER2-expressing cell proliferation is inhibited and HER2 is dephosphorylated or HER2 signal is inhibited by inhibiting energy metabolism (preferably, glucose metabolism, etc.) or by mimicking energy metabolism inhibition. Examples of "HER2 signal" include proliferation signal, antiapoptotic signal, infiltrative signal, metastasis signal or the like for the cancer cell.

Thus, energy metabolism inhibitors and energy metabolism inhibitor mimetics are useful as prophylactic/therapeutic agents for HER2-expressing cancer, and can be used as safe drugs such as a prophylactic/therapeutic agent for, for example, cancer (e.g., brain tumor, pituitary adenoma, glioma, acoustic neurinoma, retina sarcoma, thyroid cancer, pharynx cancer, larynx cancer, tongue cancer, thymoma, mesothelioma, breast cancer, lung cancer, non-small cell lung cancer, small cell lung cancer, stomach cancer, esophagus cancer, duodenal cancer, colorectal cancer, colon cancer, rectal cancer, liver cancer, hepatocarcinoma, pancreatic cancer, pancreatic endocrine tumor, bile duct cancer, gallbladder cancer, penis cancer, kidney cancer, renal pelvis cancer, ureteral cancer, renal cell cancer, testicular tumor, prostate cancer, bladder cancer, vulva cancer, uterus cancer, cervix uteri cancer, corpus uteri cancer, uterine sarcoma, trophoblastic disease, vaginal cancer, ovary cancer, ovarian germ cell tumor, skin cancer, malignant melanoma, mycosis fungoides, basal cell tumor, soft tissue sarcoma, malignant lymphoma, Hodgkin's disease, myelodysplastic syndrome, multiple myeloma, leukemia, acute myeloid leukemia, chronic myeloid leukemia, acute lymphocytic leukemia, chronic lymphocytic leukemia, adult T-cell leukemia, chronic myeloproliferative disease, pancreatic endocrine tumor, fibrous histiocytoma, leiomyoma, rhabdomyoma, cancer of unknown primary or the like, preferably breast cancer or the like), atherosclerosis, angiogenesis (e.g., angiogenesis associated with the growth of solid tumor and sarcoma, angiogenesis associated with tumor metastasis, angiogenesis associated with diabetic retinopathy, etc.), viral disease (e.g., HIV infection, etc.), rheumatoid arthritis, and further as metastasis/recurrence suppressors for cancer and pro-apoptotic agents for cancer cells.

The energy metabolism inhibitor and the energy metabolism inhibitor mimetic can be formulated as needed according to a routine procedure.

The energy metabolism inhibitor and the energy metabolism inhibitor mimetic show stronger proliferation inhibition action against HER2-expressing cancer and thus exert a good clinical effect, for example, upon administration to a patient expressing HER2 and having increased glucose metabolism for preventing/treating HER2-expressing cancer. As a result, treatment effects for this patient can be enhanced while reducing unnecessary dose and risk of adverse effect.

A patient expressing HER2 and having increased glucose metabolism may be, for example, a patient expressing HER2 (preferably, a protein containing an amino acid sequence identical to or substantially identical to the amino acid sequence represented by SEQ ID NO: 1, or a partial peptide thereof) and who has increased glucose metabolism as determined by a known method, for example, a patient who has been confirmed of the presence of cancer by PET (Positron Emission Tomography), a patient whose blood flow to cancer is confirmed by a method such as angiography or MRI (magnetic resonance imaging).

The energy metabolism inhibitor and the energy metabolism inhibitor mimetic may be used in conjunction with a hormone therapeutic drug, an anticancer drug (e.g., a chemotherapeutic agent, an immunotherapeutic drug or a drug that inhibits the effect of a cell growth factor and its receptor) or the like (hereinafter, simply referred to as a concomitant drug).

Examples of "hormone therapeutic drugs" include fosfestrol, diethylstilbestrol, chlorotrianisene, medroxyprogesterone acetate, megestrol acetate, chlormadinone acetate, cyproterone acetate, danazol, dienogest, asoprisnil, allylestrenol, gestrinone, nomegestrol, tadenan, mepartricin, raloxifene, ormeloxifene, levormeloxifene, antiestrogen (e.g., tamoxifen citrate, toremifene citrate, etc.), ER down-regulator (e.g., fulvestrant, etc.), human menopausal gonadotropin, follicle stimulating hormone, pill, mepitiostane, testrolactone, aminoglutethimide, LH-RH agonist (e.g., goserelin acetate, buserelin, leuprorelin, etc.), droloxifene, epitiostanol, ethynylestradiol sulfonate, aromatase inhibitor (e.g., fadrozole hydrochloride, anastrozole, letrozole, exemestane, vorozole, formestan, etc.), antiandrogen (e.g., flutamide, bicalutamide, nilutamide, etc.), 5α-reductase inhibitor (e.g., finasteride, dutasteride, epristeride, etc.), adrenal corticosteroid (e.g., dexamethasone, prednisolone, betamethasone, triamcinolone, etc.), androgen synthesis inhibitor (e.g., abiraterone, etc.), and drugs that slow down retinoid and retinoid metabolism (e.g., liarozole, etc.). Preferably, it is an LH-RH agonist (e.g., goserelin acetate, buserelin, leuprorelin, etc.).

Examples of "chemotherapeutic agents" include alkylating agents, antimetabolites, anticancer antibiotics and plant-derived anticancer drugs.

Examples of "alkylating agents" include nitrogen mustard, nitrogen mustard-N-oxide hydrochloride, chlorambutyl, cyclophosphamide, ifosfamide, thiotepa, carboquone, improsulfan tosilate, busulfan, nimustine hydrochloride, mitobronitol, melphalan, dacarbazine, ranimustine, estramustine sodium phosphate, triethylenemelamine, carmustine, lomustine, streptozocin, pipobroman, etoglucid, carboplatin, cisplatin, miboplatin, nedaplatin, oxaliplatin, altretamine, ambamustine, dibrospidium hydrochloride, fotemustine, prednimustine, pumitepa, ribomustine, temozolomide, threosulfan, trofosfamide, zinostatinstimalamer, adozelesin, cystemustine and bizelesin.

Examples of "antimetabolites" include mercaptopurine, 6-mercaptopurineriboside, thioinosin, methotrexate, enocitabine, cytarabine, cytarabine ocfosfate, ancitabine hydrochloride, 5-FU drugs (e.g., fluorouracil, tegafur, UFT, doxifluridine, carmofur, galocitabine, emitefur, etc.), aminopterin, leucovorin calcium, tabloid, butocin, folinate calcium, levofolinate calcium, cladribine, emitefur, fludarabine, gemcitabine, hydroxycarbamide, pentostatin, piritrexim, iodo-deoxyuridine, mitoguazone, tiazofurine and ambamustine.

Examples of "anticancer antibiotics" include actinomycin D, actinomycin C, mitomycin C, chromomycin A3, bleomycin hydrochloride, bleomycin sulfate, peplomycin sulfate, daunorubicin hydrochloride, doxorubicin hydrochloride, aclarubicin hydrochloride, pirarubicin hydrochloride, epirubicin hydrochloride, neocarzinostatin, mithramycin, sarkomycin, carzinophilin, mitotane, zorubicin hydrochloride, mitoxantrone hydrochloride and idarubicin hydrochloride.

Examples of "plant-derived anticancer drugs" include etoposide, etoposide phosphate, vinblastine sulfate, vincristine sulfate, vindesine sulfate, teniposide, paclitaxel, docetaxel and vinorelbine.

Examples of "immunotherapeutic drugs (BRM)" include picibanil, krestin, sizofiran, lentinan, ubenimex, interferon, interleukin, macrophage colony-stimulating factor, granulocyte colony-stimulating factor, erythropoietin, lymphotoxin, BCG vaccine, Corynebacterium parvum, levamisole, polysacharide-K and procodazol.

Examples of "cell growth factors" as in "drugs that inhibit effects of cell growth factors and receptors thereof" include any substance that promotes cell proliferation, usually, a peptide having a molecular weight of 20,000 or less, a factor that exerts the effect at a low concentration upon binding with the receptor, specific examples being (1) EGF (epidermal growth factor) or a substance having a substantially identical activity to EGF (e.g., EGF, heregulin (HER2 ligand), etc.), (2) insulin or a substance having a substantially identical activity to insulin (e.g., insulin, IGF (insulin-like growth factor)-1, IGF-2, etc.), (3) FGF (fibroblast growth factor) or a substance having a substantially identical activity to FGF (e.g., acidic FGF, basic FGF, KGF (keratinocyte growth factor), FGF-10, etc.), and (4) other cell growth factors (e.g., CSF (colony stimulating factor), EPO (erythropoietin), IL-2 (interleukin-2), NGF (nerve growth factor), PDGF (platelet-derived growth factor), TGFβ (transforming growth factor β), HGF (hepatocyte growth factor), VEGF (vascular endothelial growth factor), etc.).

Examples of "receptors of cell growth factors" include any receptor having an ability to bind with the cell growth factors mentioned above, specific example being, an EGF receptor, a heregulin receptor (HER2), an insulin receptor, an IGF receptor, an FGF receptor-1 or an FGF receptor-2.

Examples of "drugs that inhibit the effect of the cell growth factor" include trastuzumab (Herceptin^{™}; HER2 antibody), imatinib mesylate, ZD1839 or cetuximab, antibody for VEGF (e.g., bevacizumab), antibody for VEGF receptor, gefitinib and erlotinib.

Apart from the drugs mentioned above, L-asparaginase, aceglatone, procarbazine hydrochloride, cobalt protoporphyrin complex salt, mercury hematoporphyrin sodium, topoisomerase I inhibitors (e.g., irinotecan, topotecan, etc.), topoisomerase II inhibitors (e.g.,sobuzoxane, etc.), differentiation inducers (e.g., retinoid, vitamins D, etc.), angiogenesis inhibitors (e.g., thalidomide, SU11248, etc.), α-blocker (e.g., tamsulosin hydrochloride, naftopidil, urapidil, alfuzosin, terazocin, prazosin, silodosin, etc.), serine-threonine kinase inhibitors, endothelin receptor antagonists (e.g., atrasentan, etc.), proteasome inhibitors (e.g., bortezomib, etc.), Hsp90 inhibitors (e.g., 17-AAG, etc.), spironolactone, minoxidil, 11 α-hydroxyprogesterone, bone resorption inhibition/metastasis suppressors (e.g., zoledronic acid, alendronic acid, pamidronic acid, etidronic acid, ibandronic acid, clodronic acid) and the like can also be used.

Above all, concomitant drugs are preferably an LH-RH agonist (e.g., acetic acid goserelin, buserelin, leuprorelin, etc.), trastuzumab (HER2 antibody) or the like.

Timings of administration of the energy metabolism inhibitor and the concomitant drug are not limited, and they may be administered to the subject of administration at the same time or with time difference. The given dose may be determined appropriately based on the clinically-used dose. Moreover, the combination ratio of the energy metabolism inhibitor and the concomitant drug can be determined appropriately according to the subject to be administered, the administration route, the target disease, the symptoms, the combination and the like.

### (2) Method for screening prophylactic/therapeutic agent for HER2-expressing cancer, comprising bringing test compound into contact with cell to determine intracellular energy metabolism

In another aspect, the present invention also provides a method for screening a prophylactic/therapeutic agent for HER2-expressing cancer, comprising bringing a test compound into contact with a cell to determine intracellular energy metabolism.

The "cell" brought into contact with the test compound may be, for example, an animal cell, an insect cell or the like. Preferably, an animal cell, preferably a cultured human cancer cell or the like is used.

The animal cell used can be, for example, monkey cell COS-7, Vero, Chinese hamster cell CHO, dhfr gene-deficient Chinese hamster cell CHO, mouse L cell, mouse AtT-20 cell, mouse myeloma cell, mouse ATDC5 cell, rat GH3 cell, human FL cell, cultured human cancer cell (e.g., human breast cancer cell (e.g., BT-474, SK-BR-3, etc.), human lung cancer cell (e.g., A549, etc.), human colorectal cancer cell (e.g., HT-29, HCT-116, etc.), human pancreatic cancer cell (e.g., AsPC-1, MIA-PaCa-2, etc.), human ovary cancer cell (e.g., OV-CAR-3, CaOV-3, etc.), human kidney cancer (e.g., ACHN, etc.), etc.) or the like.

The insect cell can be, for example, Mamestra brassicae larva-derived cell line (Spodoptera frugiperda cell; Sf cell), Trichoplusia ni midgut-derived MG1 cell, Trichoplusia ni egg-derived High Five TM cell, Mamestra brassicae-derived cell, Estigmena acrea-derived cell, silk worm-derived cell line (Bombyx mori N cell; BmN cell) or the like. The Sf cell used may be, for example, Sf9 cell (ATCC CRL1711), Sf21 cell (In Vivo, 13, 213-217, (1977)) or the like.

When culturing the animal cell mentioned above, the medium used may be, for example, an MEM medium containing about 5-20% fetal bovine serum (Science, Vol. 122, 501 (1952)), a DMEM medium (Virology, Vol. 8, 396 (1959)), an RPMI 1640 medium (The Journal of the American Medical Association Vol. 199, 519 (1967)), a 199 medium (Proceeding of the Society for the Biological Medicine, Vol. 73, 1 (1950)) or the like. Preferably, pH is about 6-8. Culture is usually conducted at about 30°C -40°C for about 15-60 hours, if necessary, with aeration and agitation.

When culturing the insect cell mentioned above, the medium used may be Grace's Insect Medium (Grace, T.C.C., Nature, 195, 788 (1962)) appropriately added with an additive such as an inactivated 10% bovine serum. Preferably, pH of the medium is about 6.2-6.4. Culture is usually conducted at about 27°C for about 3-5 days, if necessary, with aeration and agitation.

The test compound used can be, for example, a synthetic compound, a peptide, protein, an antibody, a non-peptide compound, a fermented product, a cell extract, a plant extract, an animal tissue extract, plasma or the like.

Examples of the screening method include the following methods
(i) The activities of inhibiting energy metabolism (a) in the case of culturing the HER2-expressing cell and (b) in the case of culturing the HER2-expressing cell in the presence of the test compound, are determined and compared to select a compound having an energy metabolism inhibiting activity.
   (i-1) A specific example includes a method comprising determining and comparing the intracellular ATP amounts (a-1) in the case of culturing the HER2-expressing cell and (b-1) in the case of culturing the HER2-expressing cell in the presence of the test compound.
      The intracellular ATP amounts are determined by employing a known method, for example, fluorescent ATP determination (Journal of Immunologycal Methds. Vol. 160, pp. 81-88, 1993) or a method similar thereto.
   (i-2) A specific example includes a method comprising determining and comparing the mitochondrial redox activities (a-1) in the case of culturing the HER2-expressing cell and (b-1) in the case of culturing the HER2-expressing cell in the presence of the test compound.
      The mitochondrial redox activities are determined by employing a known method, for example, MTT determination (Journal of Immunologycal Methds. Vol. 65, pp. 55-63, 1983) or a method similar thereto.
      HER2-expressing cell and HER2-nonexpressing cell are seeded at about 1,000-100,000 cells/well and cultured for about 24-120 hours.
      The HER2-expressing cell used may be, for example, an "HER2-expressing cancer" cell mentioned above.
      For example, compounds that inhibit the energy metabolism inhibiting activity in the case of (b) above by about 20% or more, preferably 30% or more, more preferably about 50% or more than the energy metabolism inhibiting activity in the case of (a) above are selected as compounds that inhibit the energy metabolism.
      The culture mentioned above may be conducted by decreasing the glucose concentration or decreasing the oxygen concentration of the medium.
      In addition, for the compounds obtained in (i) above, cell proliferative activities (c) in the case of culturing the HER2-nonexpressing cell and (d) in the case of culturing the HER2-nonexpressing cell in the presence of the compound are determined and compared to eliminate compounds having nonspecific proliferation inhibition effects. For example, test compounds that have cell proliferative activities in the case of (d) above reduced by about 20% or more, preferably 30% or more, more preferably about 50% or more than the cell proliferative activity in the case of (c) above are selected as compounds having a proliferation inhibiting effect specific to HER2-expressing cell.
      Cell proliferation is determined employing a known method, for example, Sulphorhodamine-B (SRB) staining method (Journal of National Cancer Institute. Vol. 82, pp. 1107-1112, 1990), ELISA method for BrdU incorporation (Journal of Immunology Methods. Vol. 85, pp. 169-179, 1985) or the like.
      HER2-expressing cells and HER2-nonexpressing cells are seeded at about 1,000-100,000 cells/well and cultured for about 24-120 hours.
      The HER2-expressing cell used may be, for example, an "HER2-expressing cancer" cell mentioned above or the like.
(ii) AMPK protein activities (e) in the case of culturing the HER2-expressing cell and (f) in the case of culturing the HER2-expressing cell in the presence of the test compound are determined and compared to select compounds that activate AMPK protein (mimetic of energy metabolism inhibitor).
   (ii-1) A specific example includes a method comprising determining and comparing AMPK protein phosphorylations (e-1) in the case of culturing the HER2-expressing cell and (f-1) in the case of culturing the HER2-expressing cell in the presence of the test compound.
   (ii-2) A specific example includes a method comprising determining and comparing the phosphorylations of AMPK substrate protein (e-2) in the case of culturing the HER2-expressing cell and (f-2) in the case of culturing the HER2-expressing cell in the presence of the test compound.

Examples of the AMPK substrate proteins include p70 S6K protein, ACC protein and the like (Journal of Cell Science. Vol. 117, pp. 5479-5487, 2004).

The phosphorylation of the AMPK protein and the phosphorylation of the AMPK substrate protein are detected according a known method. For example, at the end of culture, an SDS sample buffer is added directly or after the extraction procedure (e.g., extraction using an RIPA buffer, etc.) to lyse the cell. The resulting cell lysate is separated by an SDS-PAGE method, and subjected to western blotting method using an antibody specific to the phosphorylation of the AMPK protein or an antibody specific to the phosphorylation of the AMPK substrate protein.

The HER2-expressing cells are seeded at about 1,000-100,000 cells/well and cultured for about 2-24 hours.

The HER2-expressing cell used is, for example, the "HER2-expressing cancer" cell described above.

For example, compounds that increase the activity of AMPK protein in the case of (f) above by about 20% or more, preferably 30% or more, more preferably about 50% or more than the activity of AMPK protein in the case of (e) above are selected as compounds that activate the AMPK protein (mimetics of energy metabolism inhibitor).

Since the compounds obtained in (i) and (ii) above have an activity of inhibiting energy metabolism or an activity of mimicking the energy metabolism inhibition (e.g., AMPK activating effect), and have an effect of selectively inhibiting proliferation of the HER2-expressing cell, they are safe and less toxic compounds specific to HER2-expressing cancer. Therefore, these compounds can orally or parenterally be administered as a prophylactic/therapeutic agent for HER2-expressing cancer, for example, to a human or a warm-blooded animal (e.g., mouse, rat, rabbit, sheep, pig, cow, horse, avian, cat, dog, monkey, chimpanzee, etc.).

The given dose of the compound or the salt thereof differs depending on its action, target disease, subject of administration, administration route or the like. For example, when the compound is administered orally for the purpose of treating breast cancer to an adult (with a weight of 60 kg), the compound or the salt thereof is generally given for about 0.1-100 mg/day, preferably about 1.0-50 mg/day, more preferably about 1.0-20 mg/day. For parenteral administration, the given dose of the compound or the salt thereof differs depending on the subject of administration, target disease or the like. For example, when the compound is administered in the form of an injectable agent for the purpose of treating breast cancer to an adult (with a weight of 60 kg), the dose of the compound or the salt thereof is generally given for about 0.01-30 mg/day, preferably about 0.1-20 mg/day, more preferably about 0.1-10 mg/day to the neoplastic lesion conveniently by injection. For other animals, a dose corresponding to the dose for the weight of 60 kg can be administered.

### (3) Method for preventing/treating HER2-expressing cancer, comprising inhibiting energy metabolism or mimicking energy metabolism inhibition

In another aspect, the present invention also provides a method for preventing/treating HER2-expressing cancer, comprising inhibiting energy metabolism of the cell or mimicking energy metabolism inhibition of the cell.

Energy metabolism of a cell is directly or indirectly associated with ATP production, a transporter, AMP-activated protein kinase or the like, and thus "inhibiting energy metabolism" typically includes, for example, inhibiting ATP production, inhibiting a transporter activity or the like.

In a preferred aspect, energy metabolism is directly associated with ATP production, which is related to, for example, glucose metabolism. Glucose metabolism includes those involved in a mitochondrial activity and those involved in glycolysis. The mitochondrial activity includes those associated with an electron transport system and those associated with a TCA cycle. In addition, those associated with an electron transport system include those associated with at least one selected from respiratory chain complexes I, II, III, IV and V.

Mimicking energy metabolism inhibition includes inducing physiologic response of a cell caused by inhibition or starvation of energy metabolism (e.g., phosphorylation and dephosphorylation of a protein, a change in active oxygen production, a change in an enzymatic activity, a change in mRNA transcription-translation, a change in protein degradation and synthesis, etc.) through an action of, for example, a synthetic compound, a peptide, protein, an antibody, a non-peptide compound, a fermented product, a cell extract, a plant extract, an animal tissue extract, plasma or the like, without directly inhibiting energy metabolism of the cell. A preferable example includes activating AMP-activated protein kinase by administering a substance having an effect of activating AMP-activated protein kinase to a cell or a subject.

The method for preventing/treating HER2-expressing cancer according to the present invention comprises, for example, administering an effective amount of the energy metabolism inhibitor or the energy metabolism inhibitor mimetic described in (1) above to a subject (for example, a mammal, preferably human). Alternatively, the method comprises, for example, administering an effective amount of a compound obtained by the screening method described in (2) above to a subject (e.g., a mammal, preferably, human). The scope of the present invention, however, is not limited by these specific aspects, and inhibiting energy metabolism of a cell of interest by providing a compound or conditions that result in an effect of directly or indirectly inhibiting intracellular energy metabolism on the cell of interest is also within the scope of the present invention.

In the present specification and the sequence listing, the bases and the amino acids may be indicated by codes, following the codes of the IUPAC-IUB Commission on Biochemical Nomenclature or codes commonly used in the field of the invention. Examples are indicated below. Moreover, if there is an enantiomer present for an amino acid, it is an L-form unless indicated otherwise.
DNA: Deoxyribonucleic acid
cDNA: Complementary deoxyribonucleic acid
A: Adenine
T: Thymine
G: Guanine
C: Cytosine
RNA: Ribonucleic acid
mRNA: Messenger ribonucleic acid
dATP: Deoxyadenosine triphosphate
dTTP: Deoxythymidine triphosphate
dGTP: Deoxyguanosine triphosphate
dCTP: Deoxycytidine triphosphate
ATP: Adenosine triphosphate
EDTA: Ethylenediaminetetraacetate
SDS: Sodium dodecylsulphate
Gly: Glycine
Ala: Alanine
Val: Valine
Leu: Leucine
Ile: Isoleucine
Ser: Serine
Thr: Threonine
Cys: Cysteine
Met: Methionine
Glu: Glutamic acid
Asp: Aspartic acid
Lys: Lysine
Arg: Arginine
His: Histidine
Phe: Phenylalanine
Tyr: Tyrosine
Trp: Tryptophan
Pro: Proline
Asn: Asparagine
Gln: Glutamine
pGlu: Pyroglutamic acid
Sec: Selenocysteine

Sequences of the present specification represent the following.
[SEQ ID NO: 1]
   represents an amino acid sequence of human HER2.
[SEQ ID NO: 2]
   represents a nucleotide sequence of cDNA coding for human HER2.
[SEQ ID NO: 3]
   represents an amino acid sequence of mouse HER2.
[SEQ ID NO: 4]
   represents a nucleotide sequence of cDNA coding for mouse HER2.
[SEQ ID NO: 5]
   represents an amino acid sequence of rat HER2.
[SEQ ID NO: 6]
   represents a nucleotide sequence of cDNA coding for rat HER2.

### EXAMPLES

The present invention should not be limited by the following examples.

### EXAMPLE 1: Inhibition of HER2-expressing cell proliferation by energy metabolism inhibition

BT-474 breast cancer cell (purchased from ATCC) was used as a human HER2-expressing cell and MDA-MB-231 breast cancer cell (purchased from ATCC) was used as a HER2-nonexpressing cell. These cells were seeded into a 96-multiwell plate (BT-474: 3.0 x 10⁴ cells/well, MDA-MB-231: 1.5 x 10⁴ cells/well), and cultured overnight in a carbon dioxide gas incubator (5% CO₂, 37°C). A 4-fold dilution series of a mitochondrial respiratory chain complex-inhibiting compound (complex I: rotenone) was added starting from a final concentration of 10 µmol/L in 10 steps, and further cultured for five days. At the end of culture, the broth containing rotenone was removed, the cells were washed with a phosphoric acid buffer, immobilized with 5% trichloroacetic acid solution, and added with 0.4% (W/V) sulphorhodamine-B (SRB) dye solution (dissolved in 1% acetic acid) for staining the cellular protein. Subsequently, the SRB dye solution was removed from the plate and the plate was washed with a 1% acetic acid solution. Then, 100 µL 10 mM Tris buffer solution was added for extraction and dye dissolution, and absorbance at an absorption wavelength of 550 nm was measured to determine the cellular quantity as a protein amount (Journal of National Cancer Institute. Vol. 82, pp. 1107-1112, 1990). Assuming that the absorbance of the well without rotenone (control) is 100%, the percentage of the absorbance of the well added with rotenone was determined to calculate the compound concentration (IC₅₀) value necessary for suppressing the remaining cellular quantity to 50% of the control.

As a result, IC₅₀ value for BT-474 cell was 0.004 µmol/L while IC₅₀ value for MDA-MB-231 cell was 0.5 µmol/L.

Accordingly, rotenone was found to suppress proliferation of the HER2-expressing BT-474 cell stronger than proliferation of the HER2-nonexpressing MDA-MB-231 cell. Thus, energy metabolism inhibition of cells by inhibition of mitochondria activity was shown to result in inhibition of cell proliferation selective to the HER2-expressing cell.

### EXAMPLE 2: Suppression of HER2 signal by inhibition of energy metabolism

Human HER2-expressing human breast cancer cell line BT-474 (purchased from American Tissue Culture Collection (ATCC)) was seeded into a 24-multiwell plate (BT-474: 1.2 x 10⁵ cells/well), and cultured overnight in a carbon dioxide gas incubator (5% CO₂, 37°C). A 4-fold dilution series of a mitochondrial respiratory chain complex inhibiting compound (complex I: rotenone, complex III: antimycin, complex V: oligomycin: Wako Pure Chemical Industries) was added starting from a final concentration of 1 µmol/L in 5 steps while continuing culture. After 2 hours, the broth was removed, and the cells were lysed with an SDS sample buffer (200 µL). Western blotting analysis was performed as follows. Specifically, this cell lysate (8 µL) was separated by an SDS-PAGE method (gel concentration: 7.5-15%) (Analytical Biochemistry, No. 166, pp. 368-379, 1987), and transferred to a nitrocellulose membrane (BioRad). Then, an antibody specific to phosphorylated HER2 (Cell signaling technology, Code# 2244), an anti-phosphorylated Akt antibody specific to downstream antiapoptotic signal transducing molecule Akt (Cell signaling technology, Code# 9271) and an anti-phosphorylated MAPK antibody specific to downstream proliferation signal transducing molecule MAPK (Cell signaling technology, Code# 9101) were used to detect the antibody with an ECL system (Amersham) to quantify the amount of phosphorylated protein by an image analysis.

Detection of HER2 phosphorylation was significant in the BT-474 cell of the solvent control group. In this case, suppression of HER2 phosphorylation was confirmed with rotenone, antimycin and oligomycin. Moreover, suppressions of Akt and MAPK phosphorylations were also confirmed.

Accordingly, it was shown that inhibition of intracellular energy metabolism by inhibition of mitochondria activity suppresses HER2 phosphorylation and suppresses downstream signal of HER2. Along with the results from Example 1, HER2 was shown to be involved in the inhibition of HER2-expressing cell proliferation by energy inhibition.

### EXAMPLE 3: Suppression of HER2 phosphorylation by suppression of aerobic metabolism (suppression of oxygen supply)

Human HER2-expressing human cancer cell line BT-474 (purchased from ATCC) was seeded into a 24-multiwell plate (1.2 x 10⁵ cells/well), and cultured overnight in a carbon dioxide gas incubator (20% O₂, 5% CO₂, 37°C). The plate was transferred to a carbon dioxide gas incubator (7% O₂, 5% CO₂, 37°C), and an SDS sample buffer (200 µL) was added to the wells over time to lyse the cells. This cell lysate (8 µL) was separated by an SDS-PAGE method (gel concentration: 7.5-15%) (Shagger et al., Analytical Biochemistry, No. 166, pp. 368-379, 1987). Then, similar to Example 1, western blotting analysis was performed to detect phosphorylated HER2, phosphorylated Akt and phosphorylated MAPK.

As can be appreciated from Figure 1, although HER2 phosphorylation for BT-474 cell was significant under usual culture conditions (20% O₂, 5% CO₂, 37°C), time-dependent inhibition of HER2 phosphorylation was confirmed by low-oxygen treatment (7% O₂, 5% CO₂, 37°C).

Thus, inhibition of energy metabolism by low oxygen was shown to suppress HER2 phosphorylation.

### EXAMPLE 4: Inhibition of HER2 phosphorylation by glucose-usage inhibition

HER2-expressing human cancer cell line SK-BR-3 (purchased from ATCC) was seeded into a 24-multiwell plate (4.0 x 10⁵ cells/well) and cultured overnight in a carbon dioxide gas incubator (5% CO₂, 37°C). Then, the medium was exchanged for a medium with a glucose content of 0.1 mg/mL and cultured for another 2 hours. A glucose antimetabolite 2-deoxyglucose was added to a final concentration of 5 mg/mL. Two hours later, the broth was removed and the cells were lysed with an SDS sample buffer (200 µL). This cell lysate (8 µL) was separated by an SDS-PAGE method (gel concentration: 7.5-15%) (Shagger et al., Analytical Biochemistry, No. 166, pp. 368-379, 1987). Then, western blotting analysis was performed in the same manner as in Example 1 to detect phosphorylated HER2, phosphorylated Akt and phosphorylated MAPK.

HER2, Akt and MAPK phosphorylations were significant for SK-BR-3 cell without the addition of 2-deoxyglucose (control), and thus addition of 2-deoxyglucose suppressed phosphorylation of each protein.

Accordingly, inhibition of energy metabolism by inhibition of glycolysis was shown to suppress HER2 phosphorylation and its downstream signal.

### EXAMPLE 5: Suppression of HER2 phosphorylation by AMPK activation

AMPK acts as a sensor of intracellular energy metabolism, and is known to be activated by inhibition of energy metabolism (by increase in ADP/ATP ratio). Whether HER2 phosphorylation and its signal are suppressed by AMPK activation (by mimicking inhibition of energy metabolism) was examined as follows.

Human HER2-expressing human cancer cell line BT-474 (purchased from ATCC) was seeded into a 24-multiwell plate (1.2 x 10⁵ cells/well) and cultured overnight in a carbon dioxide gas incubator (20% O₂, 5% CO₂, 37°C). A 3-fold dilution series of metformin that activates AMPK (The Journal of Biological Chemistry, Vol. 279, No. 42, pp. 43940-43951, 2004) was added starting from a final concentration of 15 mmol/L in 5 steps while continuing culture. Two hours later, the broth was removed and the cells were lysed with an SDS sample buffer (200 µL). This cell lysate (8 µL) was separated by an SDS-PAGE method (gel concentration: 7.5-15%) (Analytical Biochemistry, No. 166, pp. 368-379, 1987). Then, western blotting analysis was performed in the same manner as in Example 1 to quantify phosphorylated HER2, phosphorylated Akt and phosphorylated MAPK.

Detection of HER2 phosphorylation was significant for BT-474 cell of the solvent control group and thus concentration-dependent suppression of HER2 phosphorylation by addition of metformin was confirmed. Moreover, suppressions of Akt and MAPK phosphorylations were also confirmed. Accordingly, AMPK activation that mimicks energy metabolism inhibition state was shown to suppress HER2 phosphorylation and its downstream signal.

### EXAMPLE 6: Inhibition of energy metabolism and AMPK activation -Correlation with HER2 phosphorylation suppression-

Human HER2-expressing human breast cancer cell line BT-474 (purchased from American Tissue Culture Collection (ATCC)) was seeded into a 24-multiwell plate (BT-474: 1.2 x 10⁵ cells/well), and cultured overnight in a carbon dioxide gas incubator (5% CO₂, 37°C). Mitochondrial respiratory chain complex 1-inhibiting compound rotenone (Wako Pure Chemical Industries) was added in 5 steps at final concentrations of 1, 0.333, 0.111, 0.033 and 0.011 µmol/L while continuing culture. Two hours later, the broth was removed and the cells were lysed with an SDS sample buffer (200 µL). Then, this cell lysate was used to perform western blotting analysis. Specifically, this cell lysate (8 µL) was separated by an SDS-PAGE method (gel concentration: 7.5-15%) (Analytical Biochemistry, No. 166, pp. 368-379, 1987) and transferred to a nitrocellulose membrane (BioRad). Then, an antibody specific to phosphorylated HER2 (Cell signaling technology, Code# 2244), an AMPK antibody specific to AMPK (Cell signaling technology, Code# 2535) and an anti-phosphorylated AMPK antibody specific to phosphorylated AMPK (Cell signaling technology, Code# 2532) were used to detect the antibody by chemiluminescent detection with an ECL system (Amersham) to quantify an amount of phosphorylated protein with LAS-1000 plus image analysis device (FUJIFILM).

Detection of HER2 phosphorylation was significant for BT-474 cell of the solvent control group, and HER2 phosphorylation decreased dose-dependently by addition of rotenone. The amount of AMPK phosphorylation that indicates AMPK activation increased dose-dependently, inversely to the amount of HER2 phosphorylation. A quantitative change was not observed in the amount of AMPK protein itself.

Along with the results from Example 2, it was shown that when AMPK is phosphorylated by inhibition of intracellular energy metabolism, this phosphorylation correlates inversely with suppressions of phosphorylation of proliferation signal HER2 and its downstream signal.

### EXAMPLE 7: Inhibition of energy metabolism and AMPK activation -Correlation with suppression of HER2 phosphorylation-

Human HER2-expressing human breast cancer cell line BT-474 (purchased from American Tissue Culture Collection (ATCC)) was seeded into a 24-multiwell plate (BT-474: 1.2 x 10⁵ cells/well) and cultured overnight in a carbon dioxide gas incubator (5% CO₂, 37°C). As an energy metabolism inhibitor having a mechanism that differed from that of rotenone, mitochondrial respiratory chain complex III-inhibiting compound antimycin (Wako Pure Chemical Industries) was added to final concentrations of 1, 0.3, 0.1, 0.03 and 0.01 nmol/L while continuing culture. Using another plate, mitochondrial respiratory chain complex V-inhibiting compound oligomycin (Wako Pure Chemical Industries) was added to final concentrations of 100, 30, 10, 3 and 1 ng/mL while continuing culture. In addition, 2,4-dinitrophenol was added to 300, 100, 30, 10 and 3 µmol/L while continuing culture. Two hours after the addition of the agent, the broth was removed and the cells were lysed with an SDS sample buffer (200 µL). Then, this cell lysate was used to perform western blotting analysis. Specifically, this cell lysate (8 µL) was separated by an SDS-PAGE method (gel concentration: 7.5-15%) (Analytical Biochemistry, No. 166, pp. 368-379, 1987) and transferred onto a nitrocellulose membrane (BioRad). Then, an antibody specific to phosphorylated HER2 (Cell signaling technology, Code# 2244), pACC antibody specific to phosphorylated acetyl-CoA carboxylase (ACC) (Cell signaling technology, Code# 3661) and anti-phosphorylated S6K antibody specific to phosphorylated S6 kinase (S6K) (Cell signaling technology, Code# 9209) were used to detect the antibody by chemiluminescent detection with an ECL system (Amersham) to quantify an amount of phosphorylated protein with LAS-1000 plus image analysis device (FUJIFILM).

Detection of HER2 phosphorylation was significant for BT-474 cell of the solvent control group, and HER2 phosphorylation decreased dose-dependently by addition of antimycin, oligomycin and 2,4-dinitrophenol. Upon this occasion, phosphorylation of ACC as an AMPK substrate and an indicator of the activity increased dose-dependently for all compounds. Furthermore, phosphorylation of S6K known as a downstream molecule was suppressed, showing that a signal from AMPK was transduced intracellularly.

Along with the results from Examples 2 and 6, inhibition of energy metabolism was shown to suppress HER2 phosphorylation and the HER2 downstream signal, and HER2 phosphorylation was shown to correlate inversely with phosphorylation of AMPK, i.e., AMPK activity.

### EXAMPLE 8: Inhibition of HER2-expressing cell proliferation by inhibition of energy metabolism

BT-474 breast cancer cell, MDA-MB-453 breast cancer cell, A549 non-small cell lung cancer cell and LNCaP prostate cancer cell were used as human HER2-expressing cells while MDA-MB-231 breast cancer cell and A498 kidney cancer cell (all purchased from ATCC) were used as HER2-nonexpressing cells. Each of the cells used had been seeded into a 96-plate at 500cells/150 µL/well and cultured overnight. Each of the compounds, i.e., antimycin (final concentrations: 1, 0.1, 0.01 and 0.001 µmol/L), 2,4-dinitrophenol (final concentrations: 0.6, 0.2, 0.06 and 0.02 mmol/L), metformin (final concentrations: 25, 5, 1 and 0.2 mmol/L) and AICAR (final concentrations: 25, 5, 1 and 0.2 mmol/L) was diluted with a medium and added for 50 µL/well at a time to the final concentrations mentioned above. Following five days of culture at 37°C in 5% CO₂, the cells were counted using Cell Counting Kit-F (Dojindo Laboratories).

Viable cell count using Cell Counting Kit-F was carried out by the following method according to the attached instruction. The supernatant was removed from the 96-well plate after five days of culture. Cell Counting Kit-F diluted 1000-fold with an RPMI medium (phenol red-free, serum-free) was added for 100 µL/well at a time. After 30 minutes of reaction at room temperature, fluorescence (excitation wavelength; 480 nm, fluorescent wavelength; 530 nm) was determined with a fluorescent plate reader.

From the determination results, percentage of the fluorescence level in the wells added with the compound was determined assuming that the fluorescence level in the wells without the addition of the compound (control) is 100%, and the remaining cellular quantity was determined in % with respect to that of the control.

As a result, the minimum concentrations for inhibiting cell proliferation for 50% or more were as follows.

| | Antimycin | Dinitrophenol | Metformin |
|---|---|---|---|
| | (nmol/L) | (mmol/L) | (mmol/L) |
| HER2-expressing cancer | | | |
| BT474 | 1 | <0.02 | 1 |
| MB453 | 1 | <0.02 | 1 |
| A549 | 10 | <0.02 | 5 |
| LNCaP | 1 | <0.02 | 1 |
| HER2-nonexpressing cancer | | | |
| DU145 | >100 | 0.067 | >25 |
| A498 | >100 | 0.2 | >25 |
| MB231 | 10 | 0.2 | >25 |

As a result, all of the compounds were shown to act 10-100 times or more stronger against HER2-expressing cancer than against HER2-nonexpressing cancer. Along with the results from Example 1, inhibition of energy metabolism or AMPK activation was shown to suppress HER2 phosphorylation and intracellular signal transduction, inducing cell proliferation inhibition specific to HER2-expressing cancer.

### EXAMPLE 9: Specificity of HER2 signal suppression by inhibition of energy metabolism

Human EGFR-expressing human epidermal cancer cell line A431 (purchased from American Tissue Culture Collection (ATCC)) was seeded into a 24-multiwell plate (A431:6x10⁴ cells/well), and cultured overnight in a carbon dioxide gas incubator (5% CO₂, 37°C). A 4-fold dilution series of a mitochondria complex-inhibiting compound (complex I: rotenone, Wako Pure Chemical Industries) was added in 5 steps starting from a final concentration of 1 µmol/L while continuing culture. The broth was removed 2 hours later, and cells were lysed with an SDS sample buffer (200 µL). Western blotting analysis was conducted as follows. Specifically, this cell lysate (8 µL) was separated by an SDS-PAGE method (gel concentration: 7.5-15%) (Analytical Biochemistry, No. 166, pp. 368-379, 1987) and transferred onto a nitrocellulose membrane (BioRad). Subsequently, an antibody specific to phosphorylated EGFR (Cell signaling technology, Code# 2232) was used to detect an antibody with an ECL system (Amersham) to quantify the amount of phosphorylated protein by an image analysis.

Detection of EGFR phosphorylation was significant for A431 cell of the solvent control group. No suppression of EGFR phosphorylation was observed with rotenone.

EGFR is also referred to as HER1, and is a receptor tyrosine kinase that has high homology with HER2 and that belongs to the same family. Along with the results from Example 2, suppression of receptor tyrosine phosphorylation by inhibition of intracellular energy metabolism was shown to be specific to HER2.

### EXAMPLE 10: Inhibition of HER2-expressing cancer cell proliferation by AMPK activation

BT-474 breast cancer cell, MDA-MB-453 breast cancer cell and LNCaP prostate cancer cell were used as human HER2-expressing cells while MDA-MB-231 breast cancer cell and A498 kidney cancer cell (all purchased from ATCC) were used as HER2-nonexpressing cells. Each of the cells used had been seeded into a 96-well plate at 500 cells/150 µL/well and cultured overnight. Troglitazone (final concentrations: 25, 6.25 and 1.56 µmol/L) was diluted with a medium, and added for 50 µL/well at a time to the final concentrations mentioned above. Following five days of culture at 37°C in 5% CO₂, the cells were counted using Cell Counting Kit-F (Dojindo Laboratories).

Viable cell count using Cell Counting Kit-F was carried out by the following method according to the attached instruction. The supernatant was removed from the 96-well plate after five days of culture. Cell Counting Kit-F diluted 1000-fold with an RPMI medium (phenol red-free, serum-free) was added for 100 µL/well at a time. After 30 minutes of reaction at room temperature, fluorescence (excitation wavelength; 480 nm, fluorescent wavelength; 530 nm) was determined with a fluorescent plate reader.

From the determination results, percentage of the fluorescence level in the wells added with troglitazone was determined assuming that the fluorescence level in the wells without the addition of troglitazone (control) is 100%, and the remaining cellular quantity was determined in % with respect to that of the control.

As a result, for the HER2-expressing cancer cells, troglitazone at a concentration of 25 µmol/L inhibited the proliferation of BT474 cell to 50%, MB453 cell to 25% and LNCaP cell to 48%. This proliferation inhibition was dependent on the concentration. On the other hand, for the HER2-nonexpressing cancer cells, MDA-MB-231 cell was inhibited to 100% and A498 cell was inhibited to 105% at a concentration of 25 µmol/L, showing no proliferation inhibiting activity at all.

Troglitazone has been reported to activate AMPK. Along with the results from Examples 7 and 8, AMPK activation was shown to suppress HER2 phosphorylation, resulting in cell proliferation inhibition specific to HER2-expressing cancer.

### INDUSTRIAL APPLICABILITLY

Since the energy metabolism inhibitor or the energy metabolism inhibitor mimetic suppresses HER2 phosphorylation, inhibits HER2 signal and inhibits proliferation of HER2-expressing cell, it can be used, for example, as a safe drug such as a prophylactic/therapeutic agent for HER2-expressing cancer, an inhibitor for the proliferation of an HER2-expressing cell, an HER2 signal inhibitor, a dephosphorylating agent for HER2, a metastasis/recurrence suppressor for cancer, a pro-apoptotic agent for a cancer cell or a prophylactic/therapeutic agent for rheumatoid arthritis. Furthermore, the energy metabolism inhibitor or the energy metabolism inhibitor mimetic can be administered to a patient suffering from HER2-expressing cancer in order to prevent or treat the HER2-expressing cancer. A prophylactic/therapeutic agent for HER2-expressing cancer can be screened efficiently by bringing a test compound into contact with a cell to determine intracellular energy metabolism.

## Claims

1. A prophylactic/therapeutic agent for HER2-expressing cancer, comprising an energy metabolism inhibitor or an energy metabolism inhibitor mimetic.

2. A prophylactic/therapeutic agent according to Claim 1, wherein the energy metabolism inhibitor is an ATP production inhibitor.

3. A prophylactic/therapeutic agent according to Claim 2, wherein the ATP pro duction inhibitor is a glucose metabolism inhibitor.

4. A prophylactic/therapeutic agent according to Claim 3, wherein the glucose metabolism inhibitor is a mitochondrial activity inhibitor.

5. A prophylactic/therapeutic agent according to Claim 4, wherein the mitochondrial activity inhibitor is an electron transport system inhibitor.

6. A prophylactic/therapeutic agent according to Claim 5, wherein the electron transport system inhibitor is an agent that inhibits at least one selected from respiratory chain complexes I, II, III, IV and V.

7. A prophylactic/therapeutic agent according to Claim 4, wherein the mitochondrial activity inhibitor is a TCA cycle inhibitor.

8. A prophylactic/therapeutic agent according to Claim 4, wherein the mitochondrial activity inhibitor is an AMP-activated protein kinase activator.

9. A prophylactic/therapeutic agent according to Claim 3, wherein the glucose metabolism inhibitor is a glycolysis inhibitor.

10. A prophylactic/therapeutic agent according to Claim 1, wherein the energy metabolism inhibitor mimetic is an AMP-activated protein kinase activator.

11. A prophylactic/therapeutic agent for HER2-expressing cancer, comprising an energy metabolism inhibitor mimetic.

12. A prophylactic/therapeutic agent according to Claim 11, wherein the energy metabolism inhibitor mimetic is an AMP-activated protein kinase activator.

13. A prophylactic/therapeutic agent according to Claim 12, wherein the AMP-activated protein kinase activator is a mitochondrial activity inhibitor.

14. A prophylactic/therapeutic agent according to Claim 1 or 11, wherein HER2 is a protein containing an amino acid sequence identical to or substantially identical to the amino acid sequence represented by SEQ ID NO: 1, SEQ ID NO: 3 or SEQ ID NO: 5, a partial peptide thereof or a salt thereof.

15. A prophylactic/therapeutic agent according to Claim 1 or 11, which is an inhibitor for the proliferation of an HER2-expressing cell and/or an HER2 signal inhibitor.

16. A prophylactic/therapeutic agent according to Claim 15, wherein the signal is at least one selected from a proliferation signal, an antiapoptotic signal, an infiltrative signal and a metastasis signal of a cancer cell.

17. A prophylactic/therapeutic agent according to Claim 1 or 11, which is a dephosphorylating agent for HER2.

18. A prophylactic/therapeutic agent according to Claim 1 or 11, which is a metastasis/recurrence suppressor for cancer or a pro-apoptotic agent for a cancer cell.

19. A prophylactic/therapeutic agent for HER2-expressing cancer, comprising a substance having an effect of inhibiting a mitochondrial respiratory chain complex and an effect of activating an AMP-activated protein kinase.

20. A method for screening a prophylactic/therapeutic agent for HER2-expressing cancer, comprising bringing a test compound into contact with a cell to determine intracellular energy metabolism.

21. A method for preventing/treating HER2-expressing cancer, comprising inhibiting energy metabolism or mimicking energy metabolism inhibition.

22. A method for preventing/treating HER2-expressing cancer, comprising administering an effective amount of the energy metabolism inhibitor or the energy metabolism inhibitor mimetic to a mammal.

23. Use of an energy metabolism inhibitor or an energy metabolism inhibitor mimetic for the manufacture of a prophylactic/therapeutic agent for HER2-expressing cancer.

24. A method for preventing/treating HER2-expressing cancer, comprising administering the energy metabolism inhibitor or the energy metabolism inhibitor mimetic to a patient expressing HER2 and having increased glucose metabolism.


SEQUENCE LISTING

<110> Takeda Pharmaceutical Company Limited

<120> Prophylactic/therapeutic agent for HER2-expressing cancer

<130> G07-0131

<140> PCT/JP2006/313035
<141> 2006-06-23

<150> JP2005-184483
<151> 2005-06-24

<160> 6

<210> 1
<211> 1255
<212> PRT
<213> Homo sapience

<400> 1

<210> 2
<211> 3765
<212> DNA
<213> Homo sapience

<400> 2
atggagctgg cggccttgtg ccgctggggg ctcctcctcg ccctcttgcc ccccggagcc      60

gcgagcaccc aagtgtgcac cggcacagac atgaagctgc ggctccctgc cagtcccgag 120
acccacctgg acatgctccg ccacctctac cagggctgcc aggtggtgca gggaaacctg 180
gaactcacct acctgcccac caatgccagc ctgtccttcc tgcaggatat ccaggaggtg 240
cagggctacg tgctcatcgc tcacaaccaa gtgaggcagg tcccactgca gaggctgcgg 300
attgtgcgag gcacccagct ctttgaggac aactatgccc tggccgtgct agacaatgga 360
gacccgctga acaataccac ccctgtcaca ggggcctccc caggaggcct gcgggagctg 420
cagcttcgaa gcctcacaga gatcttgaaa ggaggggtct tgatccagcg gaacccccag 480
ctctgctacc aggacacgat tttgtggaag gacatcttcc acaagaacaa ccagctggct 540
ctcacactga tagacaccaa ccgctctcgg gcctgccacc cctgttctcc gatgtgtaag 600
ggctcccgct gctggggaga gagttctgag gattgtcaga gcctgacgcg cactgtctgt 660
gccggtggct gtgcccgctg caaggggcca ctgcccactg actgctgcca tgagcagtgt 720
gctgccggct gcacgggccc caagcactct gactgcctgg cctgcctcca cttcaaccac 780
agtggcatct gtgagctgca ctgcccagcc ctggtcacct acaacacaga cacgtttgag 840
tccatgccca atcccgaggg ccggtataca ttcggcgcca gctgtgtgac tgcctgtccc 900
tacaactacc tttctacgga cgtgggatcc tgcaccctcg tctgccccct gcacaaccaa 960
gaggtgacag cagaggatgg aacacagcgg tgtgagaagt gcagcaagcc ctgtgcccga 1020
gtgtgctatg gtctgggcat ggagcacttg cgagaggtga gggcagttac cagtgccaat 1080
atccaggagt ttgctggctg caagaagatc tttgggagcc tggcatttct gccggagagc 1140
tttgatgggg acccagcctc caacactgcc ccgctccagc cagagcagct ccaagtgttt 1200
gagactctgg aagagatcac aggttaccta tacatctcag catggccgga cagcctgcct 1260
gacctcagcg tcttccagaa cctgcaagta atccggggac gaattctgca caatggcgcc 1320
tactcgctga ccctgcaagg gctgggcatc agctggctgg ggctgcgctc actgagggaa 1380
ctgggcagtg gactggccct catccaccat aacacccacc tctgcttcgt gcacacggtg 1440
ccctgggacc agctctttcg gaacccgcac caagctctgc tccacactgc caaccggcca 1500
gaggacgagt gtgtgggcga gggcctggcc tgccaccagc tgtgcgcccg agggcactgc 1560
tggggtccag ggcccaccca gtgtgtcaac tgcagccagt tccttcgggg ccaggagtgc 1620
gtggaggaat gccgagtact gcaggggctc cccagggagt atgtgaatgc caggcactgt 1680
ttgccgtgcc accctgagtg tcagccccag aatggctcag tgacctgttt tggaccggag 1740
gctgaccagt gtgtggcctg tgcccactat aaggaccctc ccttctgcgt ggcccgctgc 1800
cccagcggtg tgaaacctga cctctcctac atgcccatct ggaagtttcc agatgaggag 1860
ggcgcatgcc agccttgccc catcaactgc acccactcct gtgtggacct ggatgacaag 1920
ggctgccccg ccgagcagag agccagccct ctgacgtcca tcatctctgc ggtggttggc 1980
attctgctgg tcgtggtctt gggggtggtc tttgggatcc tcatcaagcg acggcagcag 2040
aagatccgga agtacacgat gcggagactg ctgcaggaaa cggagctggt ggagccgctg 2100
acacctagcg gagcgatgcc caaccaggcg cagatgcgga tcctgaaaga gacggagctg 2160
aggaaggtga aggtgcttgg atctggcgct tttggcacag tctacaaggg catctggatc 2220
cctgatgggg agaatgtgaa aattccagtg gccatcaaag tgttgaggga aaacacatcc 2280
cccaaagcca acaaagaaat cttagacgaa gcatacgtga tggctggtgt gggctcccca 2340
tatgtctccc gccttctggg catctgcctg acatccacgg tgcagctggt gacacagctt 2400
atgccctatg gctgcctctt agaccatgtc cgggaaaacc gcggacgcct gggctcccag 2460
gacctgctga actggtgtat gcagattgcc aaggggatga gctacctgga ggatgtgcgg 2520
ctcgtacaca gggacttggc cgctcggaac gtgctggtca agagtcccaa ccatgtcaaa 2580
attacagact tcgggctggc tcggctgctg gacattgacg agacagagta ccatgcagat 2640
gggggcaagg tgcccatcaa gtggatggcg ctggagtcca ttctccgccg gcggttcacc 2700
caccagagtg atgtgtggag ttatggtgtg actgtgtggg agctgatgac ttttggggcc 2760
aaaccttacg atgggatccc agcccgggag atccctgacc tgctggaaaa gggggagcgg 2820
ctgccccagc cccccatctg caccattgat gtctacatga tcatggtcaa atgttggatg 2880

attgactctg aatgtcggcc aagattccgg gagttggtgt ctgaattctc ccgcatggcc 2940
agggaccccc agcgctttgt ggtcatccag aatgaggact tgggcccagc cagtcccttg 3000
gacagcacct tctaccgctc actgctggag gacgatgaca tgggggacct ggtggatgct 3060
gaggagtatc tggtacccca gcagggcttc ttctgtccag accctgcccc gggcgctggg 3120
ggcatggtcc accacaggca ccgcagctca tctaccagga gtggcggtgg ggacctgaca 3180
ctagggctgg agccctctga agaggaggcc cccaggtctc cactggcacc ctccgaaggg 3240
gctggctccg atgtatttga tggtgacctg ggaatggggg cagccaaggg gctgcaaagc 3300
ctccccacac atgaccccag ccctctacag cggtacagtg aggaccccac agtacccctg 3360
ccctctgaga ctgatggcta cgttgccccc ctgacctgca gcccccagcc tgaatatgtg 3420
aaccagccag atgttcggcc ccagccccct tcgccccgag agggccctct gcctgctgcc 3480
cgacctgctg gtgccactct ggaaaggccc aagactctct ccccagggaa gaatggggtc 3540
gtcaaagacg tttttgcctt tgggggtgcc gtggagaacc ccgagtactt gacaccccag 3600
ggaggagctg cccctcagcc ccaccctcct cctgccttca gcccagcctt cgacaacctc 3660
tattactggg accaggaccc accagagcgg ggggctccac ccagcacctt caaagggaca 3720
cctacggcag agaacccaga gtacctgggt ctggacgtgc cagtg 3765

<210> 3
<211> 1256
<212> PRT
<213> Mus musculus

<400> 3
Met Glu Leu Ala Ala Trp Cys Arg Trp Gly Phe Leu Leu Ala Leu Leu
                5                   10                  15
Ser Pro Gly Ala Ala Gly Thr Gln Val Cys Thr Gly Thr Asp Met Lys
            20                  25                  30
Leu Arg Leu Pro Ala Ser Pro Glu Thr His Leu Asp Met Leu Arg His
        35                  40                  45
Leu Tyr Gln Gly Cys Gln Val Val Gln Gly Asn Leu Glu Leu Thr Tyr
    50                  55                  60
Leu Pro Ala Asn Ala Ser Leu Ser Phe Leu Gln Asp Ile Gln Glu Val
65                  70                  75                  80
Gln Gly Tyr Met Leu Ile Ala His Asn Arg Val Lys His Val Pro Leu
                85                  90                  95
Gln Arg Leu Arg Ile Val Arg Gly Thr Gln Leu Phe Glu Asp Lys Tyr
            100                 105                 110
Ala Leu Ala Val Leu Asp Asn Arg Asp Pro Leu Asp Asn Val Thr Thr
        115                 120                 125
Ala Ala Pro Gly Arg Thr Pro Glu Gly Leu Arg Glu Leu Gln Leu Arg
    130                 135                 140
Ser Leu Thr Glu Ile Leu Lys Gly Gly Val Leu Ile Arg Gly Asn Pro
145                 150                 155                 160
Gln Leu Cys Tyr Gln Asp Met Val Leu Trp Lys Asp Val Leu Arg Lys
                165                 170                 175
Asn Asn Gln Leu Ala Pro Val Asp Met Asp Thr Asn Arg Ser Arg Ala
            180                 185                 190
Cys Pro Pro Cys Ala Pro Thr Cys Lys Asp Asn His Cys Trp Gly Glu

        195                 200                 205
Ser Pro Glu Asp Cys Gln Ile Leu Thr Gly Thr Ile Cys Thr Ser Gly
    210                 215                 220
Cys Ala Arg Cys Lys Gly Arg Leu Pro Thr Asp Cys Cys His Glu Gln
225                 230                 235                 240
Cys Ala Ala Gly Cys Thr Gly Pro Lys His Ser Asp Cys Leu Ala Cys
                245                 250                 255
Leu His Phe Asn His Ser Gly Ile Cys Glu Leu His Cys Pro Ala Leu
            260                 265                 270
Ile Thr Tyr Asn Thr Asp Thr Phe Glu Ser Met Leu Asn Pro Glu Gly
        275                 280                 285
Arg Tyr Thr Phe Gly Ala Ser Cys Val Thr Thr Cys Pro Tyr Asn Tyr
    290                 295                 300
Leu Ser Thr Glu Val Gly Ser Cys Thr Leu Val Cys Pro Pro Asn Asn
305                 310                 315                 320
Gln Glu Val Thr Ala Glu Asp Gly Thr Gln Arg Cys Glu Lys Cys Ser
                325                 330                 335
Lys Pro Cys Ala Gly Val Cys Tyr Gly Leu Gly Met Glu His Leu Arg
            340                 345                 350
Gly Ala Arg Ala Ile Thr Ser Asp Asn Ile Gln Glu Phe Ala Gly Cys
        355                 360                 365
Lys Lys Ile Phe Gly Ser Leu Ala Phe Leu Pro Glu Ser Phe Asp Gly
    370                 375                 380
Asn Pro Ser Ser Gly Val Ala Pro Leu Lys Pro Glu His Leu Gln Val
385                 390                 395                 400
Phe Glu Thr Leu Glu Glu Ile Thr Gly Tyr Leu Tyr Ile Ser Ala Trp
                405                 410                 415
Pro Glu Ser Phe Gln Asp Leu Ser Val Phe Gln Asn Leu Arg Val Ile
            420                 425                 430
Arg Gly Arg Ile Leu His Asp Gly Ala Tyr Ser Leu Thr Leu Gln Gly
        435                 440                 445
Leu Gly Ile His Ser Leu Gly Leu Arg Ser Leu Arg Glu Leu Gly Ser
    450                 455                 460
Gly Leu Ala Leu Ile His Arg Asn Thr His Leu Cys Phe Val Asn Thr
465                 470                 475                 480
Val Pro Trp Asp Gln Leu Phe Arg Asn Pro His Gln Ala Leu Leu His
                485                 490                 495
Ser Gly Asn Arg Pro Glu Glu Ala Cys Gly Leu Glu Gly Leu Val Cys
            500                 505                 510
Asn Ser Leu Cys Ala Arg Gly His Cys Trp Gly Pro Gly Pro Thr Gln
        515                 520                 525
Cys Val Asn Cys Ser Gln Phe Leu Arg Gly Gln Glu Cys Val Glu Glu
    530                 535                 540
Cys Arg Val Trp Lys Gly Leu Pro Arg Glu Tyr Val Arg Gly Lys His
545                 550                 555                 560
Cys Leu Pro Cys His Pro Glu Cys Gln Pro Gln Asn Ser Ser Glu Thr
                565                 570                 575

Cys Tyr Gly Ser Glu Ala Asp Gln Cys Glu Ala Cys Ala His Tyr Lys
            580                 585                 590
Asp Ser Ser Ser Cys Val Ala Arg Cys Pro Ser Gly Val Lys Pro Asp
        595                 600                 605
Leu Ser Tyr Met Pro Ile Trp Lys Tyr Pro Asp Glu Glu Gly Ile Cys
    610                 615                 620
Gln Pro Cys Pro Ile Asn Cys Thr His Ser Cys Val Asp Leu Asp Glu
625                 630                 635                 640
Arg Gly Cys Pro Ala Glu Gln Arg Ala Ser Pro Val Thr Phe Ile Ile
                645                 650                 655
Ala Thr Val Val Gly Val Leu Leu Phe Leu Ile Ile Val Val Val Ile
            660                 665                 670
Gly Ile Leu Ile Lys Arg Arg Arg Gln Lys Ile Arg Lys Tyr Thr Met
        675                 680                 685
Arg Arg Leu Leu Gln Glu Thr Glu Leu Val Glu Pro Leu Thr Pro Ser
    690                 695                 700
Gly Ala Val Pro Asn Gln Ala Gln Met Arg Ile Leu Lys Glu Thr Glu
705                 710                 715                 720
Leu Arg Lys Leu Lys Val Leu Gly Ser Gly Ala Phe Gly Thr Val Tyr
                725                 730                 735
Lys Gly Ile Trp Ile Pro Asp Gly Glu Asn Val Lys Ile Pro Val Ala
            740                 745                 750
Ile Lys Val Leu Arg Glu Asn Thr Ser Pro Lys Ala Asn Lys Glu Ile
        755                 760                 765
Leu Asp Glu Ala Tyr Val Met Ala Gly Val Gly Ser Pro Tyr Val Ser
    770                 775                 780
Arg Leu Leu Gly Ile Cys Leu Thr Ser Thr Val Gln Leu Val Thr Gln
785                 790                 795                 800
Leu Met Pro Tyr Gly Cys Leu Leu Asp His Val Arg Glu His Arg Gly
                805                 810                 815
Arg Leu Gly Ser Gln Asp Leu Leu Asn Trp Cys Val Gln Ile Ala Lys
            820                 825                 830
Gly Met Ser Tyr Leu Glu Glu Val Arg Leu Val His Arg Asp Leu Ala
        835                 840                 845
Ala Arg Asn Val Leu Val Lys Ser Pro Asn His Val Lys Ile Thr Asp
    850                 855                 860
Phe Gly Leu Ala Arg Leu Leu Asp Ile Asp Glu Thr Glu Tyr His Ala
865                 870                 875                 880
Asp Gly Gly Lys Val Pro Ile Lys Trp Met Ala Leu Glu Ser Ile Leu
                885                 890                 895
Arg Arg Arg Phe Thr His Gln Ser Asp Val Trp Ser Tyr Gly Val Thr
            900                 905                 910
Val Trp Glu Leu Met Thr Phe Gly Ala Lys Pro Tyr Asp Gly Ile Pro
        915                 920                 925
Ala Arg Glu Ile Pro Asp Leu Leu Glu Lys Gly Glu Arg Leu Pro Gln
    930                 935                 940
Pro Pro Ile Cys Thr Ile Asp Val Tyr Met Ile Met Val Lys Cys Trp

945                 950                 955                 960
Met Ile Asp Ser Glu Cys Arg Pro Arg Phe Arg Glu Leu Val Ser Glu
                965                 970                 975
Phe Ser Arg Met Ala Arg Asp Pro Gln Arg Phe Val Val Ile Gln Asn
            980                 985                 990
Glu Asp Leu Gly Pro Ser Ser Pro Met Asp Ser Thr Phe Tyr Arg Ser
        995                 1000                1005
Leu Leu Glu Asp Asp Asp Met Gly Glu Leu Val Asp Ala Glu Glu Tyr
    1010                1015                1020
Leu Val Pro Gln Gln Gly Phe Phe Ser Pro Asp Pro Ala Leu Gly Thr
1025                1030                1035                1040
Gly Ser Thr Ala His Arg Arg His Arg Ser Ser Ser Ala Arg Ser Gly
                1045                1050                1055
Gly Gly Glu Leu Thr Leu Gly Leu Glu Pro Ser Glu Glu Glu Pro Pro
            1060                1065                1070
Arg Ser Pro Leu Ala Pro Ser Glu Gly Ala Gly Ser Asp Val Phe Asp
        1075                1080                1085
Gly Asp Leu Ala Val Gly Val Thr Lys Gly Leu Gln Ser Leu Ser Pro
    1090                1095                1100
His Asp Leu Ser Pro Leu Gln Arg Tyr Ser Glu Asp Pro Thr Leu Pro
1105                1110                1115                1120
Leu Pro Pro Glu Thr Asp Gly Tyr Val Ala Pro Leu Ala Cys Ser Pro
                1125                1130                1135
Gln Pro Glu Tyr Val Asn Gln Pro Glu Val Arg Pro Gln Ser Pro Leu
            1140                1145                1150
Thr Pro Glu Gly Pro Pro Pro Pro Ile Arg Pro Ala Gly Ala Thr Leu
        1155                1160                1165
Glu Arg Pro Lys Thr Leu Ser Pro Gly Lys Asn Gly Val Val Lys Asp
    1170                1175                1180
Val Phe Ala Phe Gly Gly Ala Val Glu Asn Pro Glu Tyr Leu Ala Pro
1185                1190                1195                1200
Arg Ala Gly Thr Ala Ser Gln Pro His Pro Ser Pro Ala Phe Ser Pro
                1205                1210                1215
Ala Phe Asp Asn Leu Tyr Tyr Trp Asp Gln Asn Ser Ser Glu Gln Gly
            1220                1225                1230
Pro Pro Pro Ser Thr Phe Glu Gly Thr Pro Thr Ala Glu Asn Pro Glu
        1235                1240                1245
Tyr Leu Gly Leu Asp Val Pro Val
    1250                1255

<210> 4
<211> 3768
<212> DNA
<213> Mus musculus

<400> 4
atggagctgg cggcctggtg ccgttggggg ttcctcctcg ccctcctgtc ccccggagcc      60

gcgggtaccc aagtgtgtac cggtaccgac atgaagttgc gactccctgc cagtcctgag 120
acccacctgg acatgcttcg ccacctctac cagggctgtc aggtggtgca gggcaatttg 180
gagcttacct acctgcccgc caatgccagc ctctcattcc tgcaggacat ccaggaagtc 240
cagggataca tgctcatcgc tcacaaccga gtgaaacacg tcccactgca gaggttgcgc 300
atcgtgagag ggactcagct ctttgaggac aagtatgccc tggctgtgct agacaaccga 360
gaccctttgg acaacgtcac caccgccgcc ccaggcagaa ccccagaagg gctgcgggag 420
ctgcagcttc gaagtctcac agagatcttg aagggaggag ttttgatccg tgggaaccct 480
cagctctgct accaggacat ggttttgtgg aaggatgtcc tccgtaagaa taaccagctg 540
gctcctgtcg acatggacac caatcgttcc cgggcctgtc caccttgtgc cccaacctgc 600
aaagacaatc actgttgggg tgagagtcct gaagactgtc agatcttgac tggcaccatc 660
tgtactagtg gctgtgcccg gtgcaagggc cggctgccca ctgactgttg ccatgagcag 720
tgtgctgcag gctgcacggg tcccaagcat tctgactgcc tggcctgcct ccacttcaat 780
catagtggta tctgtgagct gcactgcccg gccctcatca cctacaacac agacaccttc 840
gagtccatgc tcaaccctga gggtcgctac acctttggtg ccagctgtgt gaccacctgc 900
ccctacaact acctctccac ggaagtggga tcctgcactc tggtctgtcc cccgaacaac 960
caagaggtca cagctgagga cggaacacag cggtgtgaga aatgcagcaa gccctgtgct 1020
ggagtatgct atggtctggg catggagcac ctccgagggg cgagggccat caccagtgac 1080
aatatccagg agtttgctgg ctgcaagaag atctttggga gcctggcatt tttgccggag 1140
agctttgatg ggaacccctc ctccggcgtt gccccactga agccagagca tctccaagtg 1200
ttcgaaaccc tggaggagat cacaggttac ctatacattt cagcatggcc agagagcttc 1260
caagacctca gtgtcttcca gaaccttcgg gtcattcggg gacggattct ccatgatggt 1320
gcttactcat tgacgttgca aggcctgggg attcactcac tggggctacg ctcactgcgg 1380
gagctgggca gtggattggc tctcattcac cgcaacaccc atctctgctt tgtaaacact 1440
gtaccttggg accagctctt ccggaacccg caccaggccc tactccacag tgggaaccgg 1500
ccagaagagg catgtggtct tgagggcttg gtctgtaact cactgtgtgc ccgtgggcac 1560
tgctgggggc cagggcccac ccagtgtgtc aactgcagtc agttcctccg gggccaggag 1620
tgtgtggagg agtgccgagt atggaagggg ctccccaggg agtatgtgag gggcaagcac 1680
tgtctgccat gccaccccga gtgtcagcct caaaacagct cggagacctg ctatggatcg 1740
gaggctgacc agtgtgaggc ttgtgcccac tacaaggact catcttcctg tgtggctcgc 1800
tgccccagtg gtgtgaagcc agacctctcc tacatgccta tctggaagta cccggatgag 1860
gagggcatat gtcagccatg ccccatcaac tgcacccact catgtgtgga cctggacgaa 1920
cgaggctgcc cagcagagca gagagccagc ccagtgacat tcatcattgc aactgtggtg 1980
ggcgtcctgt tgttcctgat catagtggtg gtcattggaa tcctaatcaa acgaaggcga 2040
cagaagatcc ggaagtatac catgcgtagg ctgctgcagg agaccgagct ggtggagccg 2100
ctgacgccca gtggagctgt gcccaaccag gctcagatgc ggatcctaaa ggagacagag 2160
ctaaggaagc tgaaggtgct tgggtcagga gccttcggca ctgtctacaa gggcatctgg 2220
atcccagatg gggagaacgt gaaaatcccc gtggccatca aggtgttgag ggaaaacaca 2280
tctcctaaag ctaacaaaga aatcctagat gaagcgtacg tcatggctgg tgtgggttct 2340
ccatatgtgt cccgcctcct gggcatctgc ctgacatcca cagtgcagct ggtgacacag 2400
cttatgccct atggctgcct tctggaccat gtccgagaac accgaggtcg cttaggctcc 2460
caggacctgc tcaactggtg tgttcagatt gccaagggga tgagctacct ggaggaagtt 2520
cggcttgttc acagggacct agctgcccga aacgtgctag tcaagagtcc caaccacgtc 2580
aagattaccg acttcgggct ggcacggctg ctggacattg atgagactga ataccatgca 2640
gatgggggca aggtgcccat caagtggatg gcattggaat ctattctcag acgccggttc 2700
acccatcaga gtgatgtgtg gagctatggt gtgactgtgt gggagctgat gacctttggg 2760
gccaaacctt acgatgggat cccagctcgg gagatccctg atttgctgga gaagggagaa 2820
cgcctacctc agcctccaat ctgcaccatc gacgtctaca tgatcatggt caaatgttgg 2880

atgattgact ccgaatgtcg cccgagattc cgggagttgg tatcagaatt ctcccgtatg 2940
gcaagggacc cccagcgctt tgtggtcatc cagaacgagg acttaggccc ctccagcccc 3000
atggacagca ccttctaccg ttcactgctg gaggatgatg acatggggga gctggtcgat 3060
gctgaagagt acctggtacc ccagcaggga ttcttctccc cagaccctgc cctaggtact 3120
gggagcacag cccaccgcag acaccgcagc tcgtcggcca ggagtggcgg tggtgagctg 3180
acactgggcc tggagccctc ggaagaagag ccccccagat ctccactggc tccctccgaa 3240
ggggctggct ccgatgtgtt tgatggtgac ctggcagtgg gggtaaccaa aggactgcag 3300
agcctctctc cacatgacct cagccctcta cagcggtaca gtgaggatcc cacattacct 3360
ctgccccccg agactgatgg ctacgttgct cccctggcct gcagccccca gcccgagtat 3420
gtgaaccagc cagaggttcg gcctcagtct cccttgaccc cagagggtcc tccgcctccc 3480
atccgacctg ctggtgctac tctagaaaga cccaagactc tctctcctgg gaaaaatggg 3540
gttgtcaaag acgtttttgc ctttgggggt gctgtggaga accctgaata cttagcaccc 3600
agagcaggca ctgcctctca gccccaccct tctcctgcct tcagcccagc ctttgacaac 3660
ctctattact gggaccagaa ctcatcggag cagggtcctc caccaagtac ctttgaaggg 3720
acccccactg cagagaaccc tgagtaccta ggcctggatg tgccagta 3768

<210> 5
<211> 1260
<212> PRT
<213> Rattus norvegicus

<400> 5
Met Ile Ile Met Glu Leu Ala Ala Trp Cys Arg Trp Gly Phe Leu Leu
                5                   10                  15
Ala Leu Leu Pro Pro Gly Ile Ala Gly Thr Gln Val Cys Thr Gly Thr
            20                  25                  30
Asp Met Lys Leu Arg Leu Pro Ala Ser Pro Glu Thr His Leu Asp Met
        35                  40                  45
Leu Arg His Leu Tyr Gln Gly Cys Gln Val Val Gln Gly Asn Leu Glu
    50                  55                  60
Leu Thr Tyr Val Pro Ala Asn Ala Ser Leu Ser Phe Leu Gln Asp Ile
65                  70                  75                  80
Gln Glu Val Gln Gly Tyr Met Leu Ile Ala His Asn Gln Val Lys Arg
                85                  90                  95
Val Pro Leu Gln Arg Leu Arg Ile Val Arg Gly Thr Gln Leu Phe Glu
            100                 105                 110
Asp Lys Tyr Ala Leu Ala Val Leu Asp Asn Arg Asp Pro Gln Asp Asn
        115                 120                 125
Val Ala Ala Ser Thr Pro Gly Arg Thr Pro Glu Gly Leu Arg Glu Leu
    130                 135                 140
Gln Leu Arg Ser Leu Thr Glu Ile Leu Lys Gly Gly Val Leu Ile Arg
145                 150                 155                 160
Gly Asn Pro Gln Leu Cys Tyr Gln Asp Met Val Leu Trp Lys Asp Val
                165                 170                 175
Phe Arg Lys Asn Asn Gln Leu Ala Pro Val Asp Ile Asp Thr Asn Arg
            180                 185                 190
Ser Arg Ala Cys Pro Pro Cys Ala Pro Ala Cys Lys Asp Asn His Cys

195                 200                 205
Trp Gly Glu Ser Pro Glu Asp Cys Gln Ile Leu Thr Gly Thr Ile Cys
    210                 215                 220
Thr Ser Gly Cys Ala Arg Cys Lys Gly Arg Leu Pro Thr Asp Cys Cys
225                 230                 235                 240
His Glu Gln Cys Ala Ala Gly Cys Thr Gly Pro Lys His Ser Asp Cys
                245                 250                 255
Leu Ala Cys Leu His Phe Asn His Ser Gly Ile Cys Glu Leu His Cys
            260                 265                 270
Pro Ala Leu Val Thr Tyr Asn Thr Asp Thr Phe Glu Ser Met His Asn
        275                 280                 285
Pro Glu Gly Arg Tyr Thr Phe Gly Ala Ser Cys Val Thr Thr Cys Pro
    290                 295                 300
Tyr Asn Tyr Leu Ser Thr Glu Val Gly Ser Cys Thr Leu Val Cys Pro
305                 310                 315                 320
Pro Asn Asn Gln Glu Val Thr Ala Glu Asp Gly Thr Gln Arg Cys Glu
                325                 330                 335
Lys Cys Ser Lys Pro Cys Ala Arg Val Cys Tyr Gly Leu Gly Met Glu
            340                 345                 350
His Leu Arg Gly Ala Arg Ala Ile Thr Ser Asp Asn Val Gln Glu Phe
        355                 360                 365
Asp Gly Cys Lys Lys Ile Phe Gly Ser Leu Ala Phe Leu Pro Glu Ser
    370                 375                 380
Phe Asp Gly Asp Pro Ser Ser Gly Ile Ala Pro Leu Arg Pro Glu Gln
385                 390                 395                 400
Leu Gln Val Phe Glu Thr Leu Glu Glu Ile Thr Gly Tyr Leu Tyr Ile
                405                 410                 415
Ser Ala Trp Pro Asp Ser Leu Arg Asp Leu Ser Val Phe Gln Asn Leu
            420                 425                 430
Arg Ile Ile Arg Gly Arg Ile Leu His Asp Gly Ala Tyr Ser Leu Thr
        435                 440                 445
Leu Gln Gly Leu Gly Ile His Ser Leu Gly Leu Arg Ser Leu Arg Glu
    450                 455                 460
Leu Gly Ser Gly Leu Ala Leu Ile His Arg Asn Ala His Leu Cys Phe
465                 470                 475                 480
Val His Thr Val Pro Trp Asp Gln Leu Phe Arg Asn Pro His Gln Ala
                485                 490                 495
Leu Leu His Ser Gly Asn Arg Pro Glu Glu Asp Leu Cys Val Ser Ser
            500                 505                 510
Gly Leu Val Cys Asn Ser Leu Cys Ala His Gly His Cys Trp Gly Pro
        515                 520                 525
Gly Pro Thr Gln Cys Val Asn Cys Ser His Phe Leu Arg Gly Gln Glu
    530                 535                 540
Cys Val Glu Glu Cys Arg Val Trp Lys Gly Leu Pro Arg Glu Tyr Val
545                 550                 555                 560
Ser Asp Lys Arg Cys Leu Pro Cys His Pro Glu Cys Gln Pro Gln Asn
                565                 570                 575

Ser Ser Glu Thr Cys Phe Gly Ser Glu Ala Asp Gln Cys Ala Ala Cys
            580                 585                 590
Ala His Tyr Lys Asp Ser Ser Ser Cys Val Ala Arg Cys Pro Ser Gly
        595                 600                 605
Val Lys Pro Asp Leu Ser Tyr Met Pro Ile Trp Lys Tyr Pro Asp Glu
    610                 615                 620
Glu Gly Ile Cys Gln Pro Cys Pro Ile Asn Cys Thr His Ser Cys Val
625                 630                 635                 640
Asp Leu Asp Glu Arg Gly Cys Pro Ala Glu Gln Arg Ala Ser Pro Val
                645                 650                 655
Thr Phe Ile Ile Ala Thr Val Glu Gly Val Leu Leu Phe Leu Ile Leu
            660                 665                 670
Val Val Val Val Gly Ile Leu Ile Lys Arg Arg Arg Gln Lys Ile Arg
        675                 680                 685
Lys Tyr Thr Met Arg Arg Leu Leu Gln Glu Thr Glu Leu Val Glu Pro
    690                 695                 700
Leu Thr Pro Ser Gly Ala Met Pro Asn Gln Ala Gln Met Arg Ile Leu
705                 710                 715                 720
Lys Glu Thr Glu Leu Arg Lys Val Lys Val Leu Gly Ser Gly Ala Phe
                725                 730                 735
Gly Thr Val Tyr Lys Gly Ile Trp Ile Pro Asp Gly Glu Asn Val Lys
            740                 745                 750
Ile Pro Val Ala Ile Lys Val Leu Arg Glu Asn Thr Ser Pro Lys Ala
        755                 760                 765
Asn Lys Glu Ile Leu Asp Glu Ala Tyr Val Met Ala Gly Val Gly Ser
    770                 775                 780
Pro Tyr Val Ser Arg Leu Leu Gly Ile Cys Leu Thr Ser Thr Val Gln
785                 790                 795                 800
Leu Val Thr Gln Leu Met Pro Tyr Gly Cys Leu Leu Asp His Val Arg
                805                 810                 815
Glu His Arg Gly Arg Leu Gly Ser Gln Asp Leu Leu Asn Trp Cys Val
            820                 825                 830
Gln Ile Ala Lys Gly Met Ser Tyr Leu Glu Asp Val Arg Leu Val His
        835                 840                 845
Arg Asp Leu Ala Ala Arg Asn Val Leu Val Lys Ser Pro Asn His Val
    850                 855                 860
Lys Ile Thr Asp Phe Gly Leu Ala Arg Leu Leu Asp Ile Asp Glu Thr
865                 870                 875                 880
Glu Tyr His Ala Asp Gly Gly Lys Val Pro Ile Lys Trp Met Ala Leu
                885                 890                 895
Glu Ser Ile Leu Arg Arg Arg Phe Thr His Gln Ser Asp Val Trp Ser
            900                 905                 910
Tyr Gly Val Thr Val Trp Glu Leu Met Thr Phe Gly Ala Lys Pro Tyr
        915                 920                 925
Asp Gly Ile Pro Ala Arg Glu Ile Pro Asp Leu Leu Glu Lys Gly Glu
    930                 935                 940
Arg Leu Pro Gln Pro Pro Ile Cys Thr Ile Asp Val Tyr Met Ile Met

945                 950                 955                 960
Val Lys Cys Trp Met Ile Asp Ser Glu Cys Arg Pro Arg Phe Arg Glu
                965                 970                 975
Leu Val Ser Glu Phe Ser Arg Met Ala Arg Asp Pro Gln Arg Phe Val
            980                 985                 990
Val Ile Gln Asn Glu Asp Leu Gly Pro Ser Ser Pro Met Asp Ser Thr
        995                 1000                1005
Phe Tyr Arg Ser Leu Leu Glu Asp Asp Asp Met Gly Asp Leu Val Asp
    1010                1015                1020
Ala Glu Glu Tyr Leu Val Pro Gln Gln Gly Phe Phe Ser Pro Asp Pro
1025                1030                1035                1040
Thr Pro Gly Thr Gly Ser Thr Ala His Arg Arg His Arg Ser Ser Ser
                1045                1050                1055
Thr Arg Ser Gly Gly Gly Glu Leu Thr Leu Gly Leu Glu Pro Ser Glu
            1060                1065                1070
Glu Gly Pro Pro Arg Ser Pro Leu Ala Pro Ser Glu Gly Ala Gly Ser
        1075                1080                1085
Asp Val Phe Asp Gly Asp Leu Ala Met Gly Val Thr Lys Gly Leu Gln
    1090                1095                1100
Ser Leu Ser Pro His Asp Leu Ser Pro Leu Gln Arg Tyr Ser Glu Asp
1105                1110                1115                1120
Pro Thr Leu Pro Leu Pro Pro Glu Thr Asp Gly Tyr Val Ala Pro Leu
                1125                1130                1135
Ala Cys Ser Pro Gln Pro Glu Tyr Val Asn Gln Ser Glu Val Gln Pro
            1140                1145                1150
Gln Pro Pro Leu Thr Pro Glu Gly Pro Leu Pro Pro Val Arg Pro Ala
        1155                1160                1165
Gly Ala Thr Leu Glu Arg Pro Lys Thr Leu Ser Pro Gly Lys Asn Gly
    1170                1175                1180
Val Val Lys Asp Val Phe Ala Phe Gly Gly Ala Val Glu Asn Pro Glu
1185                1190                1195                1200
Tyr Leu Val Pro Arg Glu Gly Thr Ala Ser Pro Pro His Pro Ser Pro
                1205                1210                1215
Ala Phe Ser Pro Ala Phe Asp Asn Leu Tyr Tyr Trp Asp Gln Asn Ser
            1220                1225                1230
Ser Glu Gln Gly Pro Pro Pro Ser Asn Phe Glu Gly Thr Pro Thr Ala
        1235                1240                1245
Glu Asn Pro Glu Tyr Leu Gly Leu Asp Val Pro Val
    1250                1255                1260

<210> 6
<211> 3780
<212> DNA
<213> Rattus norvegicus

<400> 6
atgatcatca tggagctggc ggcctggtgc cgctgggggt tcctcctcgc cctcctgccc      60

cccggaatcg cgggcaccca agtgtgtacc ggcacagaca tgaagttgcg gctccctgcc 120
agtcctgaga cccacctgga catgctccgc cacctgtacc agggctgtca ggtagtgcag 180
ggcaacttgg agcttaccta cgtgcctgcc aatgccagcc tctcattcct gcaggacatc 240
caggaagttc agggttacat gctcatcgct cacaaccagg tgaagcgcgt cccactgcaa 300
aggctgcgca tcgtgagagg gacccagctc tttgaggaca agtatgccct ggctgtgcta 360
gacaaccgag atcctcagga caatgtcgcc gcctccaccc caggcagaac cccagagggg 420
ctgcgggagc tgcagcttcg aagtctcaca gagatcctga agggaggagt tttgatccgt 480
gggaaccctc agctctgcta ccaggacatg gttttgtgga aggacgtctt ccgcaagaat 540
aaccaactgg ctcctgtcga tatagacacc aatcgttccc gggcctgtcc accttgtgcc 600
cccgcctgca aagacaatca ctgttggggt gagagtccgg aagactgtca gatcttgact 660
ggcaccatct gtaccagtgg ttgtgcccgg tgcaagggcc ggctgcccac tgactgctgc 720
catgagcagt gtgccgcagg ctgcacgggc cccaagcatt ctgactgcct ggcctgcctc 780
cacttcaatc atagtggtat ctgtgagctg cactgcccag ccctcgtcac ctacaacaca 840
gacacctttg agtccatgca caaccctgag ggtcgctaca cctttggtgc cagctgcgtg 900
accacctgcc cctacaacta cctgtctacg gaagtgggat cctgcactct ggtgtgtccc 960
ccgaataacc aagaggtcac agctgaggac ggaacacagc gttgtgagaa atgcagcaag 1020
ccctgtgctc gagtgtgcta tggtctgggc atggagcacc ttcgaggggc gagggccatc 1080
accagtgaca atgtccagga gtttgatggc tgcaagaaga tctttgggag cctggcattt 1140
ttgccggaga gctttgatgg ggacccctcc tccggcattg ctccgctgag gcctgagcag 1200
ctccaagtgt tcgaaaccct ggaggagatc acaggttacc tgtacatctc agcatggcca 1260
gacagtctcc gtgacctcag tgtcttccag aaccttcgaa tcattcgggg acggattctc 1320
cacgatggcg cgtactcatt gacactgcaa ggcctgggga tccactcgct ggggctgcgc 1380
tcactgcggg agctgggcag tggattggct ctgattcacc gcaacgccca tctctgcttt 1440
gtacacactg taccttggga ccagctcttc cggaacccac atcaggccct gctccacagt 1500
gggaaccggc cggaagagga cttgtgcgtc tcgagcggct tggtctgtaa ctcactgtgt 1560
gcccacgggc actgctgggg gccagggccc acccagtgtg tcaactgcag tcatttcctt 1620
cggggccagg agtgtgtgga ggagtgccga gtatggaagg ggctcccccg ggagtatgtg 1680
agtgacaagc gctgtctgcc gtgtcacccc gagtgtcagc ctcaaaacag ctcagagacc 1740
tgctttggat cggaggctga tcagtgtgca gcctgcgccc actacaagga ctcgtcctcc 1800
tgtgtggctc gctgccccag tggtgtgaaa ccggacctct cctacatgcc catctggaag 1860
tacccggatg aggagggcat atgccagccg tgccccatca actgcaccca ctcctgtgtg 1920
gatctggatg aacgaggctg cccagcagag cagagagcca gcccggtgac attcatcatt 1980
gcaactgtag agggcgtcct gctgttcctg atcttagtgg tggtcgttgg aatcctaatc 2040
aaacgaagga gacagaagat ccggaagtat acgatgcgta ggctgctgca ggaaactgag 2100
ttagtggagc cgctgacgcc cagcggagca atgcccaacc aggctcagat gcggatccta 2160
aaagagacgg agctaaggaa ggtgaaggtg cttggatcag gagcttttgg cactgtctac 2220
aagggcatct ggatcccaga tggggagaat gtgaaaatcc ccgtggctat caaggtgttg 2280
agagaaaaca catctcctaa agccaacaaa gaaattctag atgaagcgta tgtgatggct 2340
ggtgtgggtt ctccgtatgt gtcccgcctc ctgggcatct gcctgacatc cacagtacag 2400
ctggtgacac agcttatgcc ctacggctgc cttctggacc atgtccgaga acaccgaggt 2460
cgcctaggct cccaggacct gctcaactgg tgtgttcaga ttgccaaggg gatgagctac 2520
ctggaggacg tgcggcttgt acacagggac ctggctgccc ggaatgtgct agtcaagagt 2580
cccaaccacg tcaagattac agatttcggg ctggctcggc tgctggacat tgatgagaca 2640
gagtaccatg cagatggggg caaggtgccc atcaaatgga tggcattgga atctattctc 2700
agacgccggt tcacccatca gagtgatgtg tggagctatg gagtgactgt gtgggagctg 2760
atgacttttg gggccaaacc ttacgatgga atcccagccc gggagatccc tgatttgctg 2820
gagaagggag aacgcctacc tcagcctcca atctgcacca ttgatgtcta catgattatg 2880

gtcaaatgtt ggatgattga ctctgaatgt cgcccgagat tccgggagtt ggtgtcagaa 2940
ttttcacgta tggcgaggga cccccagcgt tttgtggtca tccagaacga ggacttgggc 3000
ccatccagcc ccatggacag taccttctac cgttcactgc tggaagatga tgacatgggt 3060
gacctggtag acgctgaaga gtatctggtg ccccagcagg gattcttctc cccggaccct 3120
accccaggca ctgggagcac agcccataga aggcaccgca gctcgtccac caggagtgga 3180
ggtggtgagc tgacactggg cctggagccc tcggaagaag ggccccccag atctccactg 3240
gctccctcgg aaggggctgg ctccgatgtg tttgatggtg acctggcaat gggggtaacc 3300
aaagggctgc agagcctctc tccacatgac ctcagccctc tacagcggta cagcgaggac 3360
cccacattac ctctgccccc cgagactgat ggctatgttg ctcccctggc ctgcagcccc 3420
cagcccgagt atgtgaacca atcagaggtt cagcctcagc ctcctttaac cccagagggt 3480
cctctgcctc ctgtccggcc tgctggtgct actctagaaa gacccaagac tctctctcct 3540
gggaagaatg gggttgtcaa agacgttttt gccttcgggg gtgctgtgga gaaccctgaa 3600
tacttagtac cgagagaagg cactgcctct ccgccccacc cttctcctgc cttcagccca 3660
gcctttgaca acctctatta ctgggaccag aactcatcgg agcaggggcc tccaccaagt 3720
aactttgaag ggacccccac tgcagagaac cctgagtacc taggcctgga tgtacctgta 3780